# EUROPÄISCHE PATENTSCHRIFT

(11) **EP 3 150 155 B1**
(45) Veröffentlichungstag und Bekanntmachung des Hinweises auf die Patenterteilung: **31.07.2019**
(21) Anmeldenummer: 16190825.6
(22) Anmeldetag: 27.09.2016
(51) Int. Cl.: A61B 17/88, B01F 13/00, A61B 90/00

(54) **VORRICHTUNG UND VERFAHREN ZUM LAGERN UND MISCHEN EINES KNOCHENZEMENTS**
DEVICE AND METHOD FOR STORING AND MIXING A BONE CEMENT
DISPOSITIF ET PROCÉDÉ DE MÉLANGE ET DE STOCKAGE D'UN CIMENT À OS

(30) Priorität: 02.10.2015 DE 102015116797
(43) Veröffentlichungstag der Anmeldung: 05.04.2017
(73) Patentinhaber: Heraeus Medical GmbH, 61273 Wehrheim (DE)
(72) Erfinder: Vogt, Sebastian Dr., 99092 Erfurt (DE)
(74) Vertreter: Schultheiss & Sterzel Patentanwälte PartG mbB

(56) Entgegenhaltungen:
- DE-A1- 1 805 110
- DE-A1-102010 004 342
- DE-A1-102012 024 710
- DE-B3-102014 108 569
- US-A1- 2011 270 260

## Beschreibung

Die Erfindung betrifft eine Vorrichtung zum Mischen eines Knochenzements. Des Weiteren betrifft die Erfindung ein Verfahren zum Mischen eines Knochenzements. Ferner betrifft die Vorrichtung eine Vorrichtung und ein Verfahren zum Lagern der Ausgangskomponenten eines Knochenzements und zum Mischen der Ausgangskomponenten.

Gegenstand der Erfindung ist somit auch eine Vorrichtung zur Lagerung und Vermischung von Polymethylmethacrylat-Knochenzement und ein Verfahren zum Mischen von Polymethylmethacrylat-Knochenzement.

Polymethylmethacrylat-Knochenzemente (PMMA-Knochenzemente) gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden mit der EP 0 692 229 A1, der DE 10 2009 031 178 B3, der DE 10 2014 108 569 B3, der US 5,997,544 A, der US 6,709,149 B1, der DE 698 12 726 T2 und der US 5,588,745 A vorgeschlagen. Auch bei diesen Mischsystemen ist eine externe Vakuumquelle notwendig. Das Patent DE 10 2009 031 178 B3 und die Patentanmeldung DE 10 2012 024 710 A1 offenbaren dabei Vakuummischvorrichtungen mit einem zweiteiligen Austragskolben. Dabei wird ein zweiteiliges Kolbensystem zum Verschluss einer Full-Prepack-Zementkartusche vorgeschlagen, bei dem eine Kombination aus einem Gasdurchlässigen Sterilisationskolben und einem Gas-undurchlässigen Dichtungskolben verwendet wird.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Die WO 2011/083 095 A1 offenbart eine Mischvorrichtung für Knochenzement, bei dem ein Kolben über eine Gewindestange in einer Zementkartusche bewegt werden kann, um einen Unterdruck im Mischraum der Kartusche zu erzeugen. Ein Mischpaddel ist als Mischreinrichtung an einer Stange angeordnet und in der Stange befindet sich eine Monomerflüssigkeit als eine Komponente des Knochenzements. Nachteilig ist hieran, dass einer zusätzlichen Durchführung bedarf, durch die neben dem Mischpaddel ein zusätzliches bewegliches Teil der Vorrichtung, nämlich der Kolben, abgedichtet geführt werden muss. Die gasdichte und drucksichte Abdichtung ist bei Durchführungen mit einem gelagerten Gewinde besonders anspruchsvoll. Zudem sind beide Seiten der Kartusche durch die jeweiligen Durchführungen blockiert.

Eine Sterilisation des Innenraums ist kurz vor dem Einsatz der Vorrichtung ebenfalls nicht mehr möglich. Für eine längere Lagerung einer Monomerflüssigkeit ist das System ebenfalls ungeeignet, da weder der Kunststoff der Stange noch die aufstechbare Folie, die zur Abdeckung des Innenraums der Stange an dem nach Innen weisenden Ende aufgeklebt ist, die stark diffusionsfähige und chemisch aggressive Monomerflüssigkeit lange halten können. Bei einem frühzeitigen Kontakt des Knochenzementpulvers mit der Monomerflüssigkeit härtet das Gemisch aus und kann anschließend durch den dafür vorgesehenen Dorn am Kolben nicht mehr geöffnet werden.

Die Aufgabe der Erfindung besteht darin, die Nachteile des Stands der Technik zu überwinden. Insbesondere soll eine Vorrichtung zum Mischen von Knochenzement bereitgestellt werden, die eine einfache Bedienung ermöglicht und in der zwei Ausgangskomponenten des Knochenzements sicher gemischt werden können, ohne dass hierfür auf zusätzliche Energiespeicher oder Versorgungsleitungen zurückgegriffen werden müsste. Es soll auch eine einfach zu bedienende Vorrichtung zum Lagern und Mischen von PMMA-Knochenzement bereitgestellt werden, bei der die Ausgangskomponenten des Knochenzements möglichst lange gelagert werden können und gleichzeitig sicher zusammengeführt und gemischt werden können sollen. Eine weitere Aufgabe besteht darin, ein Verfahren zur Herstellung von Knochenzement bereitzustellen, die bevorzugt auch zur Lagerung der Ausgangskomponenten geeignet ist. Die zu entwickelnde Vorrichtung soll möglichst eine Full-Prepac-Zementiervorrichtung für Pulver-Flüssigkeits-Polymethylmethacrylat-Knochenzemente sein. Die Vorrichtung soll kostengünstig in der Herstellung sein und eine fehlerfreie Anwendung ermöglichen. Des Weiteren sollen die Vorrichtung und das Verfahren ohne Energiespeicher und ohne Anschlüsse für Strom, Druckluft oder andere Energiequellen auskommen. Bevorzugt sollte die Vorrichtung manuell und möglichst leicht bedienbar sein. Für medizinische Anwendungen soll die Vorrichtung als Wegwerfprodukt ausgelegt sein, so dass möglichst keine Metalle oder Schadstoffe verwendet werden sollten und ein möglichst weitgehender Aufbau aus Kunststoff angestrebt wird.

Ferner sollen die Nachteile der bekannten Vakuummischsysteme mit externer Vakuumquelle überwunden werden, ohne dass ein Unterdruck über lange Zeit gehalten werden muss. Die Vorrichtung soll maximal vereinfacht sein und es erlauben, zumindest einmalig einen Unterdruck in einer Zementkartusche gegenüber der umgebenden Atmosphäre zu erzeugen. Weiterhin kann es vorteilhaft sein, wenn das Vakuummischsystem in der Lage ist, einen Transfer von Monomerflüssigkeit aus einem Monomerbehälter in eine mit Zementpulver oder mit Zementpaste gefüllte Kartusche zu ermöglichen.

Des Weiteren hat die Erfindung die Aufgabe, eine einfache geschlossene Vorrichtung zu entwickeln, bei der Polymethylmethacrylat-Knochenzementpulver und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Vorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit soll ausgeschlossen sein. Die Vorrichtung soll so beschaffen sein, dass ein Transfer der Monomerflüssigkeit in das Knochenzementpulver durch Vakuum beziehungsweise Unterdruck ohne die Verwendung von externen Vakuumpumpen erfolgt. Wichtig ist dabei, dass die Vorrichtung ohne externe Energiequellen, wie Druckluft, Vakuum oder elektrischen Strom, auch unter einfachsten äußeren Bedingungen funktionsfähig und zuverlässig die Herstellung von Knochenzementteig gewährleistet. Die Vorrichtung soll ohne zusätzliche technische Ausrüstung autonom verwendungsfähig sein. Weiterhin soll die Anzahl der Bauteile der Vorrichtung so gering wie möglich sein. Auf die Integration einer separaten Pumpe in der Vorrichtung soll möglichst verzichtet werden.

Die Hauptkomponente des Polymethylmethacrylat-Knochenzements als Mischgut soll ein Pulver sein und die zweite Komponente soll in Form einer Flüssigkeit vorliegen.

Die Aufgaben der Erfindung werden gelöst durch eine Vorrichtung zum Mischen eines Knochenzements, die Vorrichtung aufweisend
zumindest eine Kartusche, wobei die Kartusche in ihrem Inneren einen zylindrischen Mischraum aufweist,
einen Austragskolben, der im zylindrischen Mischraum in Längsrichtung beweglich ist und der gegen die Wände des Mischraums abgedichtet ist,
eine Mischeinrichtung zum Durchmischen des Inhalts des Mischraums, die im Inneren des Mischraums angeordnet ist, wobei die Mischeinrichtung über einen Mischstab von außen bedienbar ist und wobei der Mischstab durch eine Durchführung in dem Austragskolben drehbar und in Längsrichtung verschiebbar durch den Austragskolben geführt ist, wobei
die Kartusche eine dem Austragskolben gegenüberliegende Austragsöffnung aufweist, wobei im Mischraum zwischen dem Austragskolben und der Austragsöffnung eine erste Komponente des Knochenzements enthalten ist, wobei eine Monomerflüssigkeit als zweite Komponente des Knochenzements durch die Austragsöffnung in den Mischraum einleitbar ist, wobei
die Vorrichtung ein Feststellelement aufweist, mit dem der Mischstab mit dem Austragskolben derart fixierbar oder fixiert ist, dass der Austragskolben mittels des Mischstabs in dem Mischraum in Längsrichtung bewegbar ist, und wobei
der Austragskolben eine gasundurchlässige Oberseite aufweist, die von der Austragsöffnung abgewandt ist, und eine gasdurchlässige und für Pulver-Partikel undurchlässige Unterseite aufweist, die der Austragsöffnung zugewandt ist, wobei die gasdurchlässige Unterseite über zumindest eine Durchführung im Austragskolben mit zumindest einer seitlichen Öffnung in der Seitenfläche des Austragskolbens verbunden ist, so dass der Mischraum gasdurchlässig mit der Umgebung der Kartusche verbunden ist, wenn die zumindest eine Öffnung in der Seitenfläche des Austragskolbens nicht durch die Innenwand des Mischraums der Kartusche verdeckt ist.

Die Richtungsbezeichnungen oben und unten beziehen sich auf die Kartusche und die gesamte Vorrichtung, wobei die Austragsöffnung der Kartusche am unteren Ende der Kartusche angeordnet ist und der Austragskolben in das obere Ende der Kartusche eingesetzt ist. Die Zylinderachse des Mischraums erstreckt sich also von oben nach unten und der Austragskolben wird von oben nach unten vorangetrieben, um den Inhalt der Kartusche (den fertig gemischten Knochenzement) durch die Austragsöffnung auszupressen.

Die Austragsöffnung wird als solche bezeichnet, da durch sie der fertig gemischte Knochenzement aus dem Mischraum auszutreiben ist, indem der Austragskolben in Richtung der Austragsöffnung getrieben wird, auch wenn die Austragsöffnung auch zum Einleiten der Monomerflüssigkeit verwendet wird.

Bevorzugt ist der Austragskolben zylinderförmig und passend zum Verschließen des zylindrischen Mischraums geformt.

Die erste Komponente des Knochenzements ist bevorzugt ein KnochenzementPulver, besonders bevorzugt ein PMMA-Knochenzementpulver. Die zweite Komponente des Knochenzements ist die Monomerflüssigkeit. Der Knochenzementteig wird hergestellt, indem das Knochenzementpulver mit der Monomerflüssigkeit innerhalb des Mischraums mit der Mischeinrichtung gemischt wird.

Die Austragsöffnung kann auf der ins Innere des Mischraums weisenden Seite als Düse ausgebildet sein, vorzugsweise als eine Düse, wie sie in dem Patent US 8 662 736 B2 beschrieben ist.

Bei erfindungsgemäßen Vorrichtungen kann vorgesehen sein, dass der Mischstab mit dem Feststellelement lösbar mit dem Austragskolben fixierbar oder fixiert ist.

Hierdurch kann die Mischeinrichtung, die mit dem Mischstab verbunden ist, auch nach dem Bewegen des Austragskolbens mit dem Mischstab bewegt werden, in dem die Fixierung des Mischstabs mit dem Austragskolben wieder gelöst wird.

Mit einer Weiterbildung der vorliegenden Erfindung wird vorgeschlagen, dass in der Austragsöffnung oder vor der Austragsöffnung ein für Pulver undurchlässiger aber für Flüssigkeiten durchlässiger Filter vorgesehen ist, der die Austragsöffnung verschließt oder abdeckt.

Dabei ist die erste Komponente des Knochenzements pulverförmig. Hierdurch wird verhindert, dass die erste Komponente des Knochenzements durch den Filter in die Zuleitung zur Monomerflüssigkeit gelangen kann, dort vorzeitig mit der Monomerflüssigkeit reagiert und aushärtet und anschließend die Zuleitung in den Mischraum verstopft. Damit wird also verhindert, dass die Vorrichtung ungewollt nicht mehr Einsatzbereit ist.

Gemäß einer Bevorzugt Ausführung kann bei erfindungsgemäßen Vorrichtungen vorgesehen sein, dass zumindest ein Rastelement an dem Austragskolben vorgesehen ist, das derart in wenigstens eine Gegenrastung an der Kartusche greift oder dazu geeignet ist derart in wenigstens eine Gegenrastung zu greifen, dass der Austragskolben nicht mehr über die zumindest eine Rastposition hinaus in Richtung von der Austragsöffnung weg bewegt werden kann, wobei vorzugsweise das zumindest eine Rastelement an einer seitlichen Mantelfläche des Austragskolbens angeordnet ist und die wenigstens eine Gegenrastung an der Innenwand des Mischraums angeordnet ist.

Besonders bevorzugt kann das zumindest eine Rastelement lösbar mit der wenigstens einen Gegenrastung rasten. Das Lösen des zumindest einen Rastelements von der wenigstens einen Gegenrastung kann manuell erfolgen, insbesondere mit Hilfe eines Bedienelements, oder durch axiale Krafteinwirkung auf den Austragskolben in Richtung der Austragsöffnung. Nach dem Lösen des zumindest einen Rastelements von der wenigstens einen Gegenrastung soll der Austragskolben in Richtung der Austragsöffnung im Mischraum beweglich sein. Ganz besonders bevorzugt soll auch dann eine Bewegung des Austragskolbens in Richtung von der Austragsöffnung weg ausgeschlossen sein.

Durch das zumindest eine Rastelement und die wenigstens eine Gegenrastung wird erreicht, dass der Austragskolben nicht mehr so weit aus der Kartusche herausgedrückt werden kann, dass eine gasdurchlässige Verbindung von der Umgebung in den Mischraum der Kartusche entstehen kann, so wie das im weiteren Text (siehe weiter unten) vorgeschlagen wird, und gegebenenfalls mit einer weiteren Rastposition auch nicht ungewollt nach oben aus der Kartusche herausgedrückt werden kann.

Bei Vorrichtungen mit zumindest einem Rastelement kann vorgesehen sein, dass wenigstens eines der zumindest einen Rastelemente über zumindest ein Bedienelement von außen bedienbar ist, wobei vorzugsweise das Bedienelement mit dem wenigstens einen der zumindest einen Rastelemente verbunden ist und über den oberen Rand der Kartusche hinausragt, wobei der obere Rand der Kartusche der Austragsöffnung gegenüber liegt, wobei vorzugsweise das Bedienelement einteilig mit dem wenigstens einen der zumindest einen Rastelemente verbunden ist.

Das Bedienelement und das Rastelement sind bevorzugt wie die Arretierungsvorrichtung einer Kartusche gemäß der DE 10 2010 052 323 A1 aufgebaut.

Durch diese Maßnahmen wird die Bedienung vereinfacht, da die Rastelemente und damit die Rastung lösbar und von außen leicht manuell bedienbar sind.

Mit der vorliegenden Erfindung ist vorgesehen, dass der Austragskolben eine gasundurchlässige Oberseite aufweist, die von der Austragsöffnung abgewandt ist, und eine gasdurchlässige und für Pulver-Partikel undurchlässige Unterseite aufweist, die der Austragsöffnung zugewandt ist, wobei die gasdurchlässige Unterseite über zumindest eine Durchführung im Austragskolben mit zumindest einer seitlichen Öffnung in der Seitenfläche des Austragskolbens verbunden ist, so dass der Mischraum gasdurchlässig mit der Umgebung der Kartusche verbunden ist, wenn die zumindest eine Öffnung in der Seitenfläche des Austragskolbens nicht durch die Innenwand des Mischraums der Kartusche verdeckt ist.

Dabei kann vorgesehen sein, dass die gasdurchlässige Unterseite des Austragskolbens mit Hilfe einer gasdurchlässigen Porenscheibe aufgebaut ist, wobei die Porenscheibe Teil des Austragskolbens ist. Die Porenscheibe ist vorzugsweise für Zementpulver undurchlässig und für Gas durchlässig.

Durch diese Maßnahmen kann der Austragskolben, wenn er nur so weit in die Kartusche eingeschoben ist, dass die zumindest eine Öffnung in der Seitenfläche des Austragskolbens nicht durch die Innenwand des Mischraums der Kartusche verdeckt ist, zur Begasung des Mischraums mit einem sterilisierenden Gas verwendet werden. Zusätzliche Bauteile oder ein aufwendiger Umbau der Vorrichtung können damit vermieden werden. Der Austragskolben verhindert auch in der Sterilisationsstellung, dass Knochenzementpulver nach außen gelangen kann.

Der Vorteil einer solchen Vorrichtung besteht darin, dass das die Vorrichtung Innen und auch das Zementpulver durch gasförmiges Ethylenoxid sterilisiert werden kann, wobei der Gasaustausch über eine Porenscheibe erfolgt, die ein Austreten von Knochenzementpulver verhindert. Durch weiteres Einschieben des Austragskolbens in die Kartusche kann diese anschließend evakuiert werden, ohne dass jedoch der für den Gasaustausch bei Sterilisation erforderliche gasdurchlässige Austragskolben unter Öffnung der Kartusche entfernt werden muss.

Ferner wird dabei vorgeschlagen, dass der Austragskolben zwischen der gasundurchlässigen Oberseite des Austragskolbens und der zumindest einen seitlichen Öffnung eine obere umlaufende Dichtung sowie zwischen der gasdurchlässigen Unterseite und der zumindest einen seitlichen Öffnung eine untere umlaufende Dichtung aufweist, die den Austragskolben gegen die Innenwand des Mischraums abdichten oder die dazu geeignet sind, den Austragskolben gegen die Innenwand des Mischraums abzudichten, wenn der Austragskolben ausreichend tief in Richtung der Austragsöffnung in die Kartusche eingeschoben ist.

Die Dichtungen liegen vorzugsweise gasdicht an der Innenwand des Mischraums an.

Hiermit wird erreicht, dass der Mischraum bis auf die zumindest eine Durchführung verschlossen ist, wenn der Austragskolben in der Sterilisationsstellung in die Kartusche eingeschoben ist. Bei der Sterilisationsstellung des Austragskolbens ist nur die untere umlaufende Dichtung in die Kartusche eingeschoben und die obere umlaufende Dichtung liegt frei, so dass der Innenraum der Kartusche, das heißt der Mischraum, durch die zumindest eine Durchführung nach außen geöffnet ist. Wenn der Austragskolben weiter in die Kartusche in Richtung der Austragsöffnung eingeschoben wird, dann liegt ab einer bestimmten Position auch die obere umlaufende Dichtung vollumfänglich an der Innenwand der Kartusche an, so dass dann der Innenraum der Kartusche nach außen durch den Austragskolben luftdicht verschlossen ist. Hierdurch ist der Austragskolben dann in der Pumpstellung und kann als manuell betriebene Pumpe zum Einsaugen der Monomerflüssigkeit verwendet werden.

Durch die genannten Maßnahmen lässt sich also der Austragskolben gleichzeitig zum pulverdichten Sterilisieren, zum Pumpen der Flüssigkeit und später zum Austreiben der Knochenzementpaste aus der Kartusche verwenden. Diese Mehrfachverwendung birgt eine Vereinfachung des Aufbaus und damit einhergehend eine geringere Anfälligkeit für Fehler und Fehlfunktionen der Vorrichtung sowie geringere Herstellungskosten.

Es kann auch vorgesehen sein, dass zumindest zwei Rastpositionen für den Austragskolben vorgesehen sind, wobei die zumindest eine seitliche Öffnung in einer ersten Rastposition offen liegt und in einer zweiten Rastposition die zumindest eine seitliche Öffnung verdeckt ist, wobei vorzugsweise in der ersten Rastposition der Austragskolben nur durch die untere umlaufende Dichtung gegen den Mischraum abgedichtet ist und in der zweiten Rastposition der Austragskolben durch die untere und obere Dichtung gegen den Mischraum abgedichtet ist.

Hierdurch wird erreicht, dass der Austragskolben in der Sterilisationsstellung nicht ungewollt aus der Kartusche rutscht und in der Pumpstellung die zumindest eine Durchführung nicht ungewollt nach außen geöffnet wird. Damit wird die Anwendbarkeit und Bedienbarkeit der Vorrichtung vereinfacht.

Bevorzugte Ausführungsformen können sich auch dadurch auszeichnen, dass an dem Mischstab ein manuell zu bedienendes Betätigungselement befestigt ist, vorzugsweise ein einhändig zu bedienendes Griffstück befestigt ist.

Hiermit lassen sich die Mischung und der Pumpvorgang leicht manuell durchführen.

Ferner kann vorgesehen sein, dass das Feststellelement eine Schraubkappe ist, die den Mischstab umschießt und die derart auf oder in ein Gegengewinde an dem Austragskolben schraubbar ist, dass sich die Schraubkappe oder die Durchführung durch den Austragskolben derart an den Mischstab presst, dass der Mischstab gegen den Austragskolben fixiert ist, oder
das Feststellelement wenigstens ein Zapfen ist, der durch zumindest eine Durchführung im Mischstab zu stecken ist oder der in zumindest eine Vertiefung im Mischstab zu stecken ist, so dass der Mischstab nicht mehr in den Mischraum einzuführen ist, wobei vorzugsweise die zumindest eine Durchführung oder die zumindest eine Vertiefung derart positioniert ist, dass die Mischeinrichtung auf der der Austragsöffnung zugewandten Unterseite des Austragskolbens anliegt, wenn der Mischstab mit dem wenigstens einen Zapfen gegen den Austragskolben fixiert ist.

Bei der Variante mit Schraubkappe kann vorgesehen sein, dass das Gewinde der Schraubkappe oder das Gegengewinde an dem Austragskolben in Längsrichtung geschlitzt ist.

Diese Feststellelemente sind einfach zu bedienen und sind ausreichend stabil, um eine Fixierung des Mischstabs gegen den Austragskolben für einen Pumpvorgang zu gewährleisten.

Es kann des Weiteren vorgesehen sein, dass das freie Volumen im Mischraum, das nicht von der ersten Komponente und dem Austragskolben eingenommen wird, wenn der Austragskolben maximal weit in Richtung von der Austragsöffnung weg in der Kartusche angeordnet ist, mindestens so groß ist wie das Volumen der einzuleitenden Monomerflüssigkeit und aller zwischen der Monomerflüssigkeit und dem Mischraum vorgesehenen Verbindungsleitungen.

Hiermit wird sichergestellt, dass der Hubraum des Austragskolbens, wenn er zum Pumpen verwendet wird, ausreichend groß ist, um Monomerflüssigkeit in ausreichender Menge durch die Verbindungsleitungen in den Mischraum zu pumpen.

Auch kann vorgesehen sein, dass der Mischstab eine Sollbruchstelle aufweist, die unmittelbar oberhalb der Oberseite des Austragskolbens oder des Feststellelements angeordnet ist, wenn der Mischstab derart weit aus dem Mischraum herausgezogen ist, dass die Mischeinrichtung an der Unterseite des Austragskolbens anliegt.

Die Oberseite des Austragskolbens ist dabei die Seite des Austragskolbens, die von der Austragsöffnung abgewandt ist. Dementsprechend ist die Unterseite des Austragskolbens die Seite des Austragskolbens, die der Austragsöffnung zugewandt ist.

Hiermit kann der Mischstab und gegebenenfalls das Griffstück abgebrochen und entfernt werden, bevor die Kartusche mit dem Austragskolben in eine Auspressvorrichtung eingesetzt wird. In der Auspressvorrichtung würde der Mischstab stören.

Die der vorliegenden Erfindung zugrundeliegenden Aufgaben werden auch gelöst durch eine solche Vorrichtung zum Lagern der Ausgangskomponenten eines Knochenzements und zum Mischen des Knochenzements, insbesondere Full-Prepacked-Zementiervorrichtung, bei der eine Verbindungsleitung vorgesehen ist, die mit der Austragsöffnung verbunden ist und die den Mischraum flüssigkeitsdurchlässig mit einem Behälter verbindet, wobei in dem Behälter ein Monomer-Behälter mit einer Monomerflüssigkeit darin einsetzbar ist oder enthalten ist, wobei der Monomer-Behälter in der Vorrichtung zu öffnen ist und die Monomerflüssigkeit durch die Verbindungsleitung in den Mischraum einzusaugen ist, indem durch eine Bewegung des Austragskolbens von der Austragsöffnung weg im Mischraum ein Unterdruck erzeugt wird.

Hiermit wird erreicht, dass die Vorrichtung auch gut zum langfristigen Lagern der Ausgangskomponenten des Knochenzements geeignet ist und, dass der Anwender nicht ohne weiteres mit den Ausgangskomponenten des Knochenzements in Berührung kommen kann.

Bei erfindungsgemäßen Vorrichtungen, die auch zum Lagern der Ausgangskomponenten geeignet sind, kann vorgesehen sein, dass in dem Behälter zumindest eine Durchführung vorgesehen ist, wobei die zumindest eine Durchführung den Behälter mit der Umgebung gasdurchlässig verbindet.

Der Behälter weist die zumindest eine Durchführung auf, damit durch die zumindest eine Durchführung Gase entweichen können, wenn Gas aus dem Mischraum gepresst wird, und Luft nachströmen kann, wenn die Monomerflüssigkeit in den Mischraum gesaugt wird. Hiermit wird sichergestellt, dass einerseits ein gutes Vakuum beziehungsweise ein guter Unterdruck im Mischraum erzeugt werden kann und dass die Monomerflüssigkeit aus dem Behälter für die Monomerflüssigkeit leicht in den Mischraum gesaugt werden kann, ohne dass zwischen der Monomerflüssigkeit und dem Behälter für die Monomerflüssigkeit ein Unterdruck beziehungsweise ein Vakuum entsteht, dass dem Fluss der Monomerflüssigkeit in den Mischraum entgegenwirkt.

Ferner kann vorgesehen sein, dass die Verbindungsleitung zwischen dem Behälter und dem Mischraum der Kartusche eine nach oben weisende Schlaufe aufweist, wobei der höchste Punkt der Schlaufe oberhalb einer Einmündung unter dem Monomer-Behälter liegt, der in dem Behälter angeordnet ist.

Bei korrekter Aufstellung der Vorrichtung kann so sichergestellt werden, dass die Monomerflüssigkeit nicht ohne Einwirkung des mit dem Austragskolben erzeugten Unterdrucks ungewollt in die Kartusche fließen kann und dort ungewollt vorzeitig mit dem Knochenzementpulver reagiert und aushärtet. Die Schlaufe wirkt somit als Siphon. Damit kann verhindert werden, dass die Monomerflüssigkeit beim Öffnen des Monomer-Behälters (der Monomer-Glasampulle) in dem Behälter bereits durch die Flüssigkeitsleitung in den Innenraum der Kartusche gelangt. Diese umgekehrt U-förmige Schlaufe der Verbindungsleitung erreicht, dass vor Bewegung des Austragskolbens als Pumpkolben die Monomerflüssigkeit im Behälter bis zur Höhe des Scheitelpunkts in der Flüssigkeitsleitung beziehungsweise Verbindungsleitung verbleibt, wodurch ein vorzeitiger Eintritt der Monomerflüssigkeit zum Zementpulver verhindert wird. Insbesondere bei hoch-viskosen Zementen kann ein vorzeitiger Kontakt von schon geringen Volumina der Monomerflüssigkeit mit dem Zementpulver zur Verklebung der Verbindungsleitung oder eines als Düse ausgebildeten Leitungsmittels, wie in US 8 662 736 B2 beschrieben, führen. Die Verbindungsleitung kann transparent oder transluzent sein, damit der Anwender den Monomertransfer visuell kontrollieren kann. Hierzu kann vorzugsweise ein Sichtfenster in der Vorrichtung beziehungsweise in einem Gehäuse der Vorrichtung vorgesehen sein, durch das die Schlaufe mit dem höchsten Scheitelpunkt zu erkennen ist.

Der Monomer-Behälter ist bevorzugt eine aufbrechbare Glasampulle, die die Monomerflüssigkeit enthält, oder ein Mehrschichtverbundfolienbeutel, der die Monomerflüssigkeit enthält, wobei bevorzugt der Mehrschichtverbundfolienbeutel ein mit Aluminium beschichteter Mehrschichtverbundfolienbeutel ist. Bevorzugt weisen die Glasampullen einen zylindrischen Ampullenkörper auf.

Glasampullen und Mehrschichtverbundfolienbeutel sind dazu in der Lage, die Monomerflüssigkeit lange zu halten, ohne dass die Monomerflüssigkeit entweichen kann. Hierdurch kann die Vorrichtung also auch dazu verwendet werden, die Ausgangskomponenten über einen langen Zeitraum zu lagern.

Bei solchen Vorrichtungen kann vorgesehen sein, dass die Vorrichtung eine Öffnungseinrichtung aufweist, mit der der Monomer-Behälter in dem Behälter zu öffnen ist, vorzugsweise ohne die Vorrichtung dabei zu öffnen oder ohne den Behälter dabei zu öffnen.

Hiermit wird verhindert, dass der Anwender beim Öffnen des Monomer-Behälters mit der Monomerflüssigkeit in Berührung kommen kann.

Des Weiteren kann vorgesehen sein, dass die Vorrichtung ein Fußteil aufweist, das mit der Kartusche verbunden ist und an dem auch der Behälter befestigt ist, wobei vorzugsweise die Kartusche lösbar mit dem Fußteil verbunden ist, besonders bevorzugt über ein Gewinde lösbar mit dem Fußteil verbunden ist.

Hiermit lässt sich die Vorrichtung leicht auf einem Tisch aufstellen und ist dadurch bequem zu bedienen. Eine Fehlbedienung durch falsche Aufstellung kann dadurch ebenfalls ausgeschlossen werden. Zudem kann das Fußteil zur Verbindung und Stabilisierung aller Elemente der Vorrichtung verwendet werden.

Die der Erfindung zugrundeliegenden Aufgaben werden ferner gelöst durch ein Verfahren zum Herstellen eines Knochenzements aus einer ersten Komponente und einer Monomer-Flüssigkeit als zweiter Komponente, insbesondere mit einer erfindungsgemäßen Vorrichtung, bei dem
ein Mischstab mit Hilfe des Feststellelements mit einem Austragskolben fixiert wird oder fixiert ist,
der Austragskolben von einer Austragsöffnung weg bewegt wird und sich dadurch ein Unterdruck in einem Mischraum bildet, wobei vorzugsweise der Austragskolben manuell mit Hilfe des Mischstabs bewegt wird,
mit dem Unterdruck eine Monomerflüssigkeit in den Mischraum gesaugt wird,
der Mischstab von dem Austragskolben gelöst wird und die erste Komponente des Knochenzements und die Monomerflüssigkeit im Mischraum mit einer Mischeinrichtung durch manuelles Bewegen des Mischstabs zu einer Knochenzementpaste gemischt wird, wobei
der Mischraum zunächst über den Austragskolben gasdurchlässig mit der Umgebung einer Kartusche verbunden ist, wobei die Vorrichtung einschließlich des Mischraums durch Evakuieren und Einleiten eines sterilisierenden Gases, insbesondere von Ethylenoxid, sterilisiert wird,
anschließend der Austragskolben in Richtung der Austragsöffnung bewegt wird, so dass die gasdurchlässigen Öffnungen im Austragskolben durch die Innenwand des Mischraums verdeckt werden, und
anschließend der Austragskolben derart tief in den Mischraum eingeschoben wird, dass Gas aus dem Mischraum durch die Austragsöffnung herausgedrückt wird, bevor der Austragskolben von der Austragsöffnung weg bewegt wird.

Zudem kann vorgesehen sein, dass nach dem Fixieren des Mischstabs der Austragskolben derart tief in den Mischraum eingeschoben wird, dass Gas aus dem Mischraum durch die Austragsöffnung herausgedrückt wird, bevor der Austragskolben von der Austragsöffnung weg bewegt wird.

Bei erfindungsgemäßen Verfahren kann vorgesehen sein, dass der Mischraum zunächst über den Austragskolben gasdurchlässig mit der Umgebung einer Kartusche verbunden ist, wobei die Vorrichtung einschließlich des Mischraums durch Evakuieren und Einleiten eines sterilisierenden Gases, insbesondere von Ethylenoxid, sterilisiert wird,
anschließend der Austragskolben in Richtung der Austragsöffnung bewegt wird, so dass die gasdurchlässigen Öffnungen im Austragskolben durch die Innenwand des Mischraums verdeckt werden, und
anschließend der Austragskolben derart tief in den Mischraum eingeschoben wird, dass Gas aus dem Mischraum durch die Austragsöffnung herausgedrückt wird, bevor der Austragskolben von der Austragsöffnung weg bewegt wird,
wobei anschließend der Monomerbehälter in dem Behälter der Vorrichtung geöffnet wird, bevor der Austragskolben von der Austragsöffnung weg bewegt wird.

Die Kartusche umfasst den Mischraum, beziehungsweise der Mischraum ist im Inneren der Kartusche angeordnet.

Beim Evakuieren des Mischraums der Kartusche ist bevorzugt der Austragskolben gegen die Kartusche mit Hilfe des zumindest einen Rastelements und der wenigstens einen Gegenrastung gesichert beziehungsweise verbunden. Der Monomerbehälter kann auch bereits unmittelbar vor dem Bewegen des Austragskolbens in Richtung der Austragsöffnung geöffnet werden. Dann wird das Gas aus dem Mischraum der Kartusche durch die Verbindungsleitung und durch die Monomerflüssigkeit in dem Behälter gedrückt. Der Behälter weist Durchführungen auf, durch die die Gase entweichen können und durch die Luft nachströmen kann, wenn die Monomerflüssigkeit in den Mischraum gesaugt wird.

Auch kann vorgesehen sein, dass der Austragskolben zumindest so weit in Richtung der Austragsöffnung in den Mischraum eingeschoben wird, dass der dabei überfahrene Hubraum mindestens so groß ist, wie das Volumen der einzuleitenden Monomerflüssigkeit und aller Verbindungsleitungen, durch die die Monomerflüssigkeit in den Mischraum geleitet wird.

Hiermit wird sichergestellt, dass mit dem Hub des Austragskolbens beim Pumpen eine ausreichende Menge der Monomerflüssigkeit in den Mischraum gesaugt werden kann, um einen Knochenzement in der gewünschten Konsistenz zu erzeugen.

Bei erfindungsgemäßen Verfahren kann vorgesehen sein, dass bei einem Einschieben des Austragskolbens in den Mischraum Gas aus dem Mischraum durch die Austragsöffnung und durch zumindest eine Durchführung in einem Behälter für die Monomerflüssigkeit aus der Vorrichtung herausgedrückt wird. Es kann alternativ oder zusätzlich auch vorgesehen sein, dass wenn die Monomerflüssigkeit in den Mischraum gesaugt wird, Luft durch zumindest eine Durchführung in einem Behälter für die Monomerflüssigkeit in den Behälter nachströmt.

Der Behälter weist die zumindest eine Durchführung auf, damit durch die zumindest eine Durchführung Gase entweichen können, wenn Gas aus dem Mischraum gepresst wird, und Luft nachströmen kann, wenn die Monomerflüssigkeit in den Mischraum gesaugt wird. Hiermit wird sichergestellt, dass einerseits ein gutes Vakuum beziehungsweise ein guter Unterdruck im Mischraum erzeugt werden kann und dass die Monomerflüssigkeit aus dem Behälter für die Monomerflüssigkeit leicht in den Mischraum gesaugt werden kann, ohne dass zwischen der Monomerflüssigkeit und dem Behälter für die Monomerflüssigkeit ein Unterdruck beziehungsweise ein Vakuum entsteht, dass dem Fluss der Monomerflüssigkeit in den Mischraum entgegenwirkt.

Des Weiteren kann vorgesehen sein, dass die Monomerflüssigkeit und die erste Komponente des Knochenzements, insbesondere die Monomerflüssigkeit und das Knochenzementpulver, im Bereich einer Einmündung für die Monomerflüssigkeit in den Mischraum, insbesondere im Bereich einer Düse, miteinander reagieren, wobei dadurch die Einmündung, insbesondere die Düse, verklebt.

Durch das Verkleben der Einmündung beziehungsweise der Düse wird ein Eintritt von gemischtem Knochenzementteig in die Verbindungsleitung der Monomerflüssigkeit verhindert.

Ferner kann vorgesehen sein, dass nach dem Durchmischen der Ausgangskomponenten des Knochenzements der Mischstab aus dem Mischraum herausgezogen wird, bis die Mischeinrichtung an der Unterseite des Austragskolbens anliegt, wobei bevorzugt der Mischstab anschließend an einer Sollbruchstelle, die dann oberhalb des Austragskolbens oder oberhalb des Feststellelements liegt, abgebrochen wird.

Schließlich kann vorgesehen sein, dass die Kartusche als letzter Schritt von der Verbindungsleitung und gegebenenfalls von dem Fußteil gelöst wird und in eine Auspressvorrichtung zum Auspressen des Knochenzements aus der Kartusche eingesetzt wird.

Der Erfindung liegt die überraschende Erkenntnis zugrunde, dass es mit der erfindungsgemäßen Vorrichtung und dem erfindungsgemäßen Verfahren gelingt, den Austragskolben sowohl zum Austragen des fertig gemischten Knochenzements zu verwenden als auch als Pumpe zum Einsaugen der Monomerflüssigkeit, wobei der gleiche Mischstab, der auch zum Bedienen der Mischeinrichtung zum Durchmischen des Inhalts des Mischraums verwendet wird gleichzeitig zum Bedienen der Pumpe beziehungsweise zum Bedienen des Austragskolbens als Pumpe verwendbar ist. Dies alles gelingt mit einem geschlossenen System, bei dem der Anwender nicht mit dem Knochenzementpulver in Berührung kommen kann und bei geeignetem Aufbau auch nicht mit der Monomerflüssigkeit in Berührung kommen kann. Mit einer Weiterbildung kann der Austragskolben auch noch als pulverdichte Durchführung zur Sterilisation des Inhalts des Mischraums verwendet werden. Hiermit wird erreicht, dass der Aufbau der Vorrichtung einfach gehalten ist und kostengünstig bleibt. Durch die Vermeidung komplexerer Strukturen, die größere Kräfte aushalten müssen, wie beispielsweise Motoren, Energiespeicher, Zuleitungen oder metallische Gewindestangen mit metallischen Lagern, kann die Vorrichtung vollständig oder zumindest sehr weitgehend aus Kunststoff aufgebaut werden, so dass die Vorrichtung als kostengünstiges Wegwerfprodukt konzipiert werden kann, was aufgrund der hygienischen Anforderungen, die an eine Vorrichtung zur Herstellung von Knochenzement aufgrund des Einsatzes im OP-Bereich gestellt werden, vorteilhaft ist.

Die Erfindung basiert auf der Idee, die ohnehin vorhandene Kartusche selbst als Pumpengehäuse zu nutzen und den Austragskolben als Pumpkolben mit dem Mischstab als Betätigungselement zu nutzen, um einen Unterdruck in der Kartusche zu erzeugen. Mit diesem Unterdruck wird die Monomerflüssigkeit aus dem geöffneten Monomerflüssigkeitsbehälter in die Kartusche über eine Verbindungsleitung in das Zementpulver gesaugt. Überraschend wurde gefunden, dass der durch manuelle axiale Aufwärtsbewegung des Austragskolbens in der Kartusche erzeugte Unterdruck ausreicht, die Monomerflüssigkeit sicher aus dem geöffneten Monomerbehälter in den Mischraum zum Zementpulver zu transferieren.

Eine beispielhafte erfindungsgemäße Vorrichtung zum Mischen von PMMA-Knochenzement und zum Lagern der Ausgangskomponenten des PMMA-Knochenzements, kann beispielsweise zusammengesetzt sein aus
a) einem zylinderförmigen Austragskolben, mit einem ersten Hohlraum (als der Durchführung des Austragskolbens) der von einer gasundurchlässigen Oberseite und einer gasdurchlässigen und für Pulver-Partikel undurchlässigen Unterseite und einer Mantelfläche begrenzt ist, wobei die Mantelfläche mindestens eine gasdurchlässige Durchbrechung besitzt, wobei durch die Oberseite des Austragskolbens, durch den ersten Hohlraum und die Unterseite des Austragskolbens ein axial beweglicher Mischstab gasdicht geführt wird,
b) einem lösbaren Feststellelement auf der Oberseite des Austragskolbens zur lösbaren Fixierung des Mischstabs, wobei vorzugsweise das Feststellelement mit dem Austragskolben unlösbar verbunden ist,
c) einem an der Mantelfläche des Austragskolbens umlaufenden Dichtungselement, das unterhalb der gasdurchlässigen Durchbrechung angeordnet ist,
d) einem an der Mantelfläche des Austragskolbens umlaufenden Dichtungselement, das oberhalb der gasdurchlässigen Durchbrechung angeordnet ist,
e) mindestens einem Rastelement an der äußeren Mantelfläche des Austragskolbens,
f) einer zylinderförmigen Kartusche, wobei eine obere Stirnseite der Kartusche mit dem Austragskolben verschlossen ist, und wobei die untere Stirnseite der Kartusche mit einem für Zementpulver undurchlässigen, aber flüssigkeitsdurchlässigen Verschluss verschlossen ist,
g) einem zweiten Hohlraum, der durch den Innenraum der mit dem Austragskolben und mit dem Verschluss begrenzten Kartusche gebildet ist, wobei in dem zweiten Hohlraum Zementpulver angeordnet ist,
h) wobei der zweite Hohlraum über einen für Pulver undurchlässigen, flüssigkeitsdurchlässigen Verschluss mit einer flüssigkeitsdurchlässigen Verbindungsleitung verbunden ist,
i) einem Monomerflüssigkeitsbehälter und ein Öffnungselement,
j) wobei die flüssigkeitsdurchlässige Verbindungsleitung mit dem Öffnungselement des Monomerflüssigkeitsbehälter verbunden ist,
k) einer Mischeinrichtung, die im zweiten Hohlraum am Ende des Mischstabs befestigt ist, und
l) einem manuell zu bedienenden Betätigungselement an dem Ende des Mischstabs außerhalb der Kartusche.

Gemäß einer Weiterbildung der vorliegenden Erfindung kann vorgesehen sein, dass während der Gassterilisation mit Ethylenoxid der Austragskolben in die Kartusche derart tief eingedrückt ist, dass die zumindest eine gasdurchlässige Öffnung in der Mantelfläche des Austragskolbens über den oberen Rand der Kartusche beziehungsweise des ersten Hohlzylinders hinausragt, wobei das untere Dichtungselement des Austragskolbens gasdicht an der Innenseite der Kartusche also am Rand des Mischraums anliegt. Nach der Sterilisation mit Ethylenoxid wird vor dem Monomertransfer der Austragskolben so in den Mischraum beziehungsweise ersten Hohlraum der Kartusche eingeschoben, dass die gasundurchlässige Oberseite des Austragskolbens tiefer als der äußere Rand der Kartusche ist, wobei das obere Dichtungselement des Austragskolbens an der Innenseite der Kartusche und den zweiten Hohlraum, also den Mischraum, gasdicht an der Oberseite der Kartusche verschließt.

Wesentlich für die Funktion der Vorrichtung kann es gemäß einer Weiterbildung der vorliegenden Erfindung auch sein, dass bei maximaler axialer Verschiebung des Austragskolbens in der Kartusche in Richtung der Austragsöffnung ein dritter Hohlraum oberhalb des Austragskolbens gebildet wird, der durch die offene Oberseite der Kartusche und die Innenfläche der Kartusche gebildet wird, wobei das Volumen des dritten Hohlraums gleich oder größer ist als die Summe der Volumina der Monomerflüssigkeit und der Verbindungsleitung.

Ein erfindungsgemäßes Verfahren zum Mischen eines Knochenzements mit einer erfindungsgemäßen Vorrichtung, wie mit der bespielhaft beschriebenen Vorrichtung kann beispielsweise durch folgende chronologische Schritte gekennzeichnet sein:
a) der Mischstab wird durch das Feststellelement am Austragskolben festgelegt,
b) durch Lösen der Rastung wird der Austragskolben axial in der Kartusche beweglich,
c) über das Betätigungselement der Mischstange wird die Mischstange mit dem daran fixierten Austragskolben nach unten in Richtung des Zementpulvers bewegt, wobei die im zweiten Hohlraum vorhandene Luft durch den Verschluss austritt,
d) die nach unten gerichtete Bewegung des Austragskolbens wird mindestens so weit fortgesetzt, bis das Volumen des dritten Hohlraums mindestens gleich der Summe der Volumina der Monomerflüssigkeit und der Verbindungsleitung ist,
e) durch Betätigen des Öffnungselements wird der Monomerflüssigkeitsbehälter geöffnet,
f) anschließend wird manuell mit dem Betätigungselement die Mischstange mit dem daran fixierten Austragskolben nach oben gezogen, wobei ein Unterdruck an der Unterseite des Austragskolbens entsteht und die Monomerflüssigkeit angesaugt wird,
g) die Monomerflüssigkeit aus dem Monomerflüssigkeitsbehälter strömt durch die Verbindungsleitung und den Verschluss in die Kartusche zum Zementpulver,
h) der Austragskolben wird durch die Rastung reversibel mit der Kartusche verbunden,
i) das Feststellelement wird gelöst,
j) der axiale bewegliche Mischstab wird manuell axial zur Vermischung des Zementpulvers mit der Monomerflüssigkeit bewegt, bis ein homogener Zementteig entstanden ist,
k) der Mischstab wird nach oben gezogen, bis die Mischeinrichtung an der Unterseite des Austragskolbens anliegt, und
l) der Mischstab wird an einer Sollbruchstelle, die oberhalb des Feststellelements des fixierten Mischstabs ausgebildet ist, abgebrochen.

Ein zweites beispielhaftes erfindungsgemäßes Verfahren ist durch folgende nacheinander ablaufende Schritte charakterisiert:
a) der Mischstab wird durch das Feststellelement am Austragskolben festgelegt,
b) durch Lösen der Rastung wird der Austragskolben axial in der Kartusche beweglich,
c) durch Betätigen des Öffnungselements wird der Monomerflüssigkeitsbehälter geöffnet,
d) über das Betätigungselement der Mischstange wird die Mischstange mit dem daran fixierten Austragskolben nach unten in Richtung des Zementpulvers bewegt, wobei die im zweiten Hohlraum vorhandene Luft durch den Verschluss in die Verbindungsleitung austritt und entweicht durch eine Öffnung im Öffnungselement in die umgebende Atmosphäre,
e) die nach unten gerichtete Bewegung des Austragskolbens wird mindestens so weit fortgesetzt, bis das Volumen des dritten Hohlraums mindestens gleich der Summe der Volumina der Monomerflüssigkeit und der Verbindungsleitung ist,
f) anschließend wird mit dem Betätigungselement die Mischstange mit dem daran fixierten Austragskolben manuell nach oben gezogen, wobei ein Unterdruck an der Unterseite des Austragskolbens entsteht und die Monomerflüssigkeit angesaugt wird,
g) die Monomerflüssigkeit strömt aus dem Monomerflüssigkeitsbehälter durch die Verbindungsleitung und den Verschluss in die Kartusche zum Zementpulver,
h) der Austragskolben wird durch die Rastung reversibel mit der Kartusche verbunden,
i) das Feststellelement wird gelöst,
j) der axial bewegliche Mischstab wird zur Vermischung des Zementpulvers mit der Monomerflüssigkeit manuell axial bewegt bis ein homogener Zementteig entstanden ist,
k) der Mischstab wird nach oben gezogen, bis die Mischeinrichtung an der Unterseite des Austragskolbens anliegt, und
l) der Mischstab wird an einer Sollbruchstelle, die oberhalb des Feststellelements ausgebildet ist, abgebrochen.

Erfindungsgemäß ist weiterhin ein Verfahren, bei dem nach dem Schritt I) der beiden vorgenannten Verfahren die Kartusche vom Fußteil gelöst wird und mit einer Auspressvorrichtung verbunden wird.

Im Folgenden werden weitere Ausführungsbeispiele der Erfindung anhand von fünf schematisch dargestellten Figuren erläutert, ohne jedoch dabei die Erfindung zu beschränken. Dabei zeigt:
- Figur 1:: eine schematische Querschnittansicht einer erfindungsgemäßen Vorrichtung zum Mischen eines Knochenzements und zum Lagern der Ausgangskomponenten;
- Figur 2:: eine schematische perspektivische Ansicht der Vorrichtung nach Figur 1;
- Figur 3:: eine schematische Querschnittansicht einer alternativen erfindungsgemäßen Vorrichtung ohne Bedienelemente zum Lösen der Rastung vor dem Pumpvorgang;
- Figur 4:: eine schematische Querschnittansicht der Vorrichtung nach Figur 3 nach dem Pumpvorgang; und
- Figur 5:: eine schematische Querschnittansicht der Vorrichtung nach den Figuren 3 und 4 nach dem Mischen des Knochenzements.

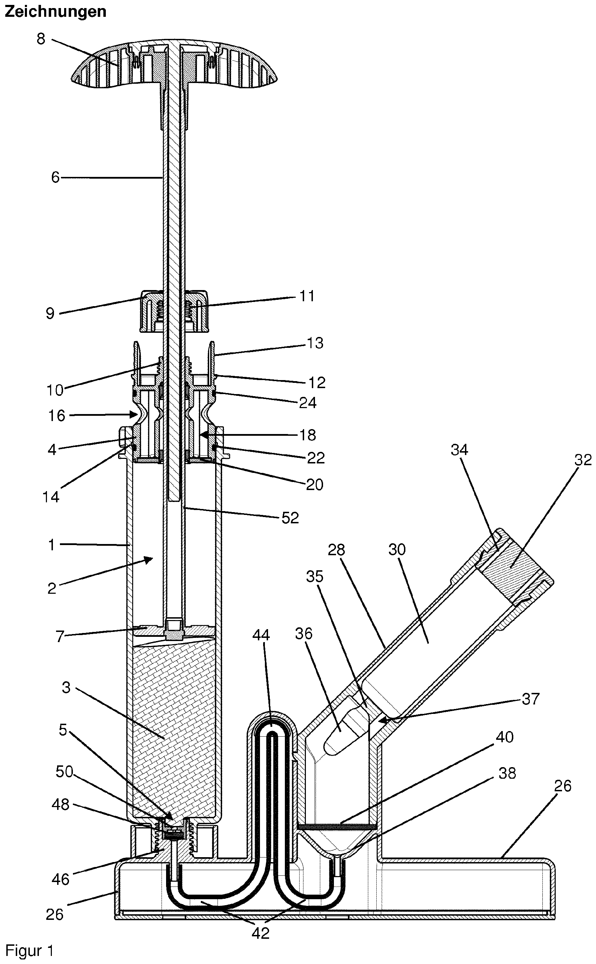

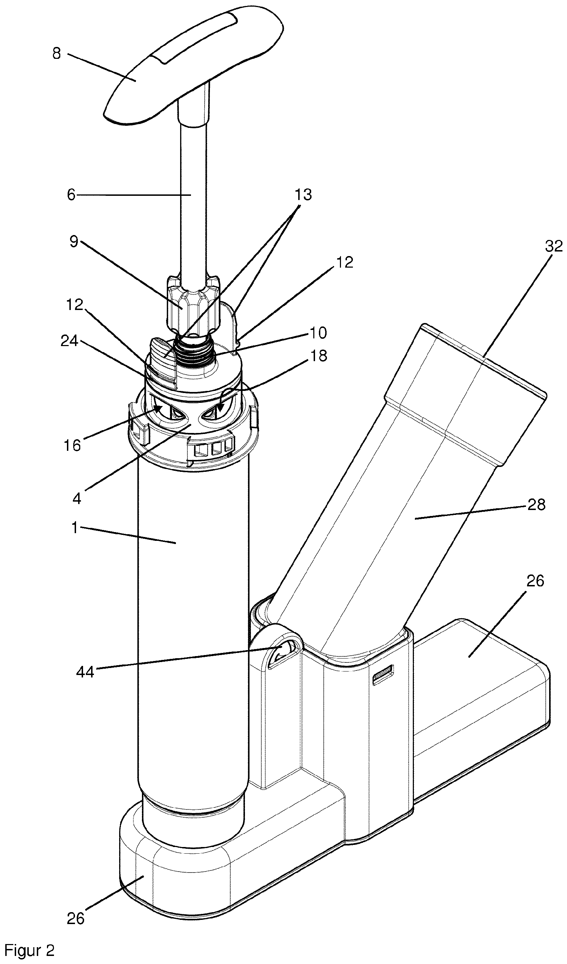

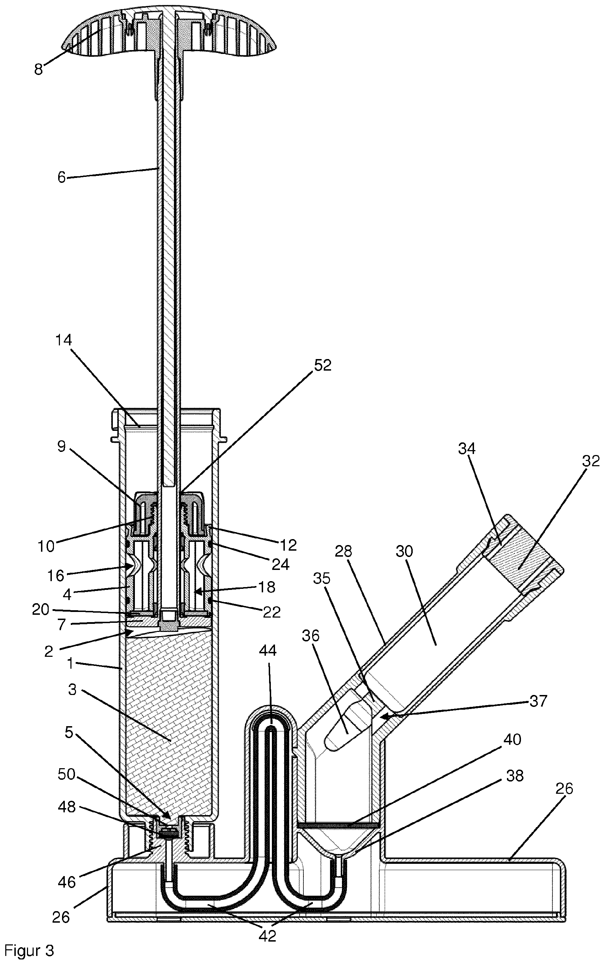

Die Figuren 1 und 2 zeigen eine erste erfindungsgemäße Vorrichtung zum Mischen von Knochenzement und zum Lagern der Ausgangskomponenten eines Knochenzements in einer Querschnittansicht (Figur 1) und in einer perspektivischen Seitenansicht (Figur 2). Die Figuren 3 bis 5 zeigen schematische Querschnittansichten einer alternativen Vorrichtung zum Mischen von Knochenzement und zum Lagern der Ausgangskomponenten eines Knochenzements, wobei sich diese lediglich dadurch von der Ausführung nach den Figuren 1 und 2 unterscheidet, dass die Rastung nicht mit Hilfe von Bedienelementen 13 von außerhalb gelöst werden kann. Aufgrund der weitgehenden Ähnlichkeit der Ausführungsbeispiele werden gleiche oder gleichartige Bauteile in beiden Figuren mit den gleichen Bezugszeichen bezeichnet, auch wenn die Bauteile sich unterscheiden können. Mit den Figuren 3 bis 5 wird der Ablauf eines erfindungsgemäßen Verfahrens erläutert. Das Verfahren kann aber in gleicher Weise ebenso ohne weiteres mit der Vorrichtung nach den Figuren 1 oder 2 durchgeführt werden, wobei die Rastung eben manuell über die Bedienelemente 13 gelöst wird und nicht durch Ausüben eines Drucks (siehe unten). Beide Vorrichtungen umfassen eine erfindungsgemäße Vorrichtung zum Mischen von Knochenzement, die als Teil der Vorrichtungen zum Mischen von Knochenzement und zum Lagern der Ausgangskomponenten eines Knochenzements aufgefasst werden muss.

Die Vorrichtung zum Mischen von Knochenzement und zum Lagern der Ausgangskomponenten eines Knochenzements in einer Querschnittansicht umfasst eine Kartusche 1 in deren Inneren ein Mischraum 2 vorgesehen ist, der seitlich durch die Wände der Kartusche 1 begrenzt ist. Die Kartusche 1 ist zylindrisch geformt und der durch die Innenwände der Kartusche 1 vorgegebene Mischraum 2 ist ebenfalls zylindrisch. Im Inneren des Mischraums 2 befindet sich ein Knochenzementpulver 3 als erste Komponente des zu erzeugenden PMMA-Knochenzements. Der Mischraum ist an der Oberseite durch einen Austragskolben 4 begrenzt und auf der gegenüberliegenden Unterseite durch eine Abschlusswand mit einer Austragsöffnung 5. Der Austragskolben 4 ist passend zu den zylindrischen Innenwänden der Kartusche 1 geformt und ist in Längsrichtung (in den Figuren 1, 3, 4 und 5 nach oben und unten) beweglich in dem Mischraum 2 beziehungsweise in der Kartusche 1 angeordnet.

Ein Mischstab 6 erstreckt sich durch eine zentrische und abgedichtete Durchführung im Austragskolben 4. Der Mischstab 6 ist in Längsrichtung linear beweglich und kann um die eigene Achse gegen den Austragskolben 4 gedreht werden, um eine Mischeinrichtung mit Mischflügeln 7 zu bewegen, die sich im Mischraum 2 befindet. Mit der Mischeinrichtung 7 und dem Mischstab 6 kann der Inhalt des Mischraums 2 manuell durchmischt werden. Hierzu ist am Mischstab 6 ein Griff 8 befestigt, mit dem der Mischstab 6 und damit die Mischeinrichtung 7 bewegt werden kann.

Um den Mischstab 6 herum ist eine Schraubkappe 9 angeordnet, die zusammen mit einer Hülse mit einem Außengewinde 10, die an dem Austragskolben 4 befestigt ist, beziehungsweise einteilig mit dem Austragskolben 4 ausgeführt ist, ein Feststellelement 9, 10 bildet, mit dem der Mischstab 6 mit dem Austragskolben 4 fixiert werden kann, indem die Schraubkappe 9 auf das Außengewinde 10 aufgeschraubt wird. Hierzu weist die Schraubkappe 9 ein zum Außengewinde 10 passendes Innengewinde 11 auf. Die Hülse mit dem Außengewinde 10 ist mehrfach in Längsrichtung geschlitzt und das Innengewinde 11 leicht konisch zusammenlaufend ausgeführt, so dass die Hülse mit dem Außengewinde 10 an den Mischstab 6 angepresst wird, wenn die Schraubkappe 9 auf das Außengewinde 10 aufgeschraubt wird. Hierdurch kann eine stabile und lösbare Fixierung des Mischstabs 6 mit dem Austragskolben 4 erreicht werden. Alternativ könnte auch ein Stift oder Bolzen (nicht gezeigt) durch eine Durchführung (nicht gezeigt) im Mischstab 6 geschoben werden, um eine Bewegung des Mischstabs 6 ins Innere des Mischraums 2 hinein zu blockieren. Weitere Ausführungen für andere Feststellelemente sind ohne weiteres denkbar.

Am Austragskolben 4 sind zwei Rastelemente 12 vorgesehen, die bei der Ausführung nach den Figuren 1 und 2 mit einem Bedienelement 13 manuell gelöst werden können, wenn sie eingerastet sind. Bei der Ausführung nach den Figuren 3 bis 5 werden die Rastelemente 12 einfach durch einen ausreichenden Druck gelöst, der mit dem fixierten Mischstab 6 auf den Austragskolben 4 und damit auf die Rastelemente 12 ausgeübt werden kann. Damit die Rastelemente 12 rasten und damit den Austragskolben 4 gegen die Kartusche 1 fixieren können, ist im Innenumfang der Kartusche eine Nut 14 als Gegenrastung 14 vorgesehen. Die Gegenrastung 14 ist in Richtung des Griffs 8 steiler ausgeführt als in Richtung der Austragsöffnung 5, so dass der Austragskolben 4 zwar tiefer in den Mischraum 2 hinein geschoben werden kann, nicht aber ohne weiteres nach oben aus dem Mischraum 2 beziehungsweise der Kartusche 1 hinaus gezogen werden kann.

Der Austragskolben 4 weist in der seitlichen Mantelfläche mehrere seitliche Öffnungen 16 auf, die ins Innere des Austragskolbens 4 führen und die mit einer Durchführung 18 verbunden sind, die zur unteren Fläche des Austragskolbens 4 führt (der zur Austragsöffnung 5 weisenden Fläche des Austragskolbens 4 führt). Dadurch verbindet die Durchführung 18 die seitlichen Öffnungen 16 des Austragskolbens 4 mit dem Mischraum 2 und somit den Mischraum 2 mit der Umgebung der Kartusche 1. Um zu verhindern, dass Knochenzementpulver 3 nach außen dringen kann, ist an der unteren Fläche des Austragskolbens 4 eine Porenscheibe 20 angeordnet, die die Durchführung 18 komplett abdeckt und die für Pulver-Partikel des Knochenzementpulvers 3 undurchlässig ist aber für Gas durchlässig ist. Hierdurch kann der Mischraum 2 evakuiert werden und es kann ein sterilisierendes Gas, wie Ethylenoxid eingespeist werden, um den Mischraum 2 und das Knochenzementpulver 3 zu sterilisieren, ohne dass das Knochenzementpulver 3 nach außen dringen kann. In der Innenwand des Austragskolbens 4 sind weitere Öffnungen in Richtung des Mischstabs 6 beziehungsweise der Durchführung für den Mischstab 6 vorgesehen, durch die das sterilisierende Gas bis zum Mischstab 6 in diesem Bereich vordringen kann. Hiermit wird erreicht, dass die Vorrichtung auch in diesem Bereich und damit vollständig sterilisiert werden kann.

Zwischen der Unterseite des Austragskolbens 4 und den seitlichen Öffnungen 16 ist eine umlaufende untere Gummi-Dichtung 22 vorgesehen, mit der der Austragskolben 4 gegen die Innenwände der Kartusche 1 abgedichtet ist. Zwischen der Oberseite des Austragskolbens 4 und den seitlichen Öffnungen 16 ist eine umlaufende obere Gummi-Dichtung 24 vorgesehen, mit der der Austragskolben 4 gegen die Innenwände der Kartusche 1 abgedichtet ist, wenn der Austragskolben 4 tief genug in die Kartusche 1 eingeschoben ist. Die Gummi-Dichtungen 22, 24 dichten gasdicht ab. Wenn nur die untere Dichtung 22 in die Kartusche 1 eingeschoben ist (wie in Figur 1 und Figur 2 gezeigt), dann kann Gas durch die Durchführung 18 von außen in den Mischraum 2 eingeleitet werden oder aus dem Mischraum 2 evakuiert werden. Wenn auch die obere Dichtung 24 an der Innenwand der Kartusche 1 anliegt, dann schließt der Austragskolben 4 den Mischraum 2 nach außen gasdicht ab. Der Austragskolben 4 kann dann als Pumpe verwendet werden, indem er mit dem Mischstab 6 bewegt wird. Hierzu muss der Mischstab 6 mit dem Austragskolben 4 mit Hilfe des Feststellelements 9, 10 fixiert sein.

Alle bisher beschriebenen Bauteile, also insbesondere die Kartusche 1, der Austragskolben 4, der Mischstab 6, die Mischeinrichtung 7, der Griff 8, die Schraubkappe 9, die Porenscheibe 20 und die Dichtungen 22, 24, bilden eine erfindungsgemäße Vorrichtung zum Mischen von Knochenzement, die als Teil einer erfindungsgemäßen Vorrichtung zum Mischen von Knochenzement und zum Lagern der Ausgangskomponenten des Knochenzements verstanden wird. Diese Teilvorrichtung ist in den Querschnittansichten nach den Figuren 1, 3, 4 und 5 auf der linken Seite (oberhalb eines Standfußes 26) dargestellt. Diese Teilvorrichtung, also die Vorrichtung zum Mischen von Knochenzement ist zwar auch zum Lagern der ersten Komponente des Knochenzements, nämlich des Knochenzementpulvers 3 geeignet, ist aber nicht dazu geeignet, die zweite Komponente des Knochenzements, nämlich eine Monomerflüssigkeit zu lagern. Die Teilvorrichtung kann dennoch einzeln verwendet werden, in dem eine Monomerflüssigkeit durch die Austragsöffnung 5 in den Mischraum 2 eingespeist wird und mit der Mischeinrichtung 7 mit dem Knochenzementpulver 3 durchmischt wird, um einen Knochenzementteig zu erzeugen.

Bei den vorliegenden Ausführungsbeispielen ist die Teilvorrichtung jedoch mit weiteren Bauteilen kombiniert, um eine erfindungsgemäße Vorrichtung zum Mischen von Knochenzement und auch zum Lagern der Ausgangskomponenten des Knochenzements zu bilden.

Die erfindungsgemäße Vorrichtung zum Mischen von Knochenzement und zum Lagern der Ausgangskomponenten des Knochenzements weist dazu ferner einen Standfuß 26 auf, auf dem die Kartusche 1 aufgeschraubt ist und gehalten wird. Der Standfuß 26 hat eine flache Unterseite und kann einfach auf einem Tisch aufgestellt werden. Zudem hält der Standfuß 26 die Vorrichtung zusammen, indem er ein Gehäuse bildet. In den Standfuß 26 ist ein elastisch verformbarer Behälter 28 zur Aufnahme einer Glasampulle 30 eingesetzt. Der Behälter 28 besteht aus Gummi oder einem anderen elastischen Kunststoff. Die Glasampulle 30 enthält eine Monomerflüssigkeit als zweite Komponente des PMMA-Knochenzements. Die Glasampulle 30 dient also als Monomerflüssigkeitsbehälter 30 der Vorrichtung. Durch die Verwendung des Materials Glas, kann die Monomerflüssigkeit sehr lange in der Vorrichtung gelagert werden, da die Monomerflüssigkeit nicht ohne weiteres durch das Glas austreten kann.

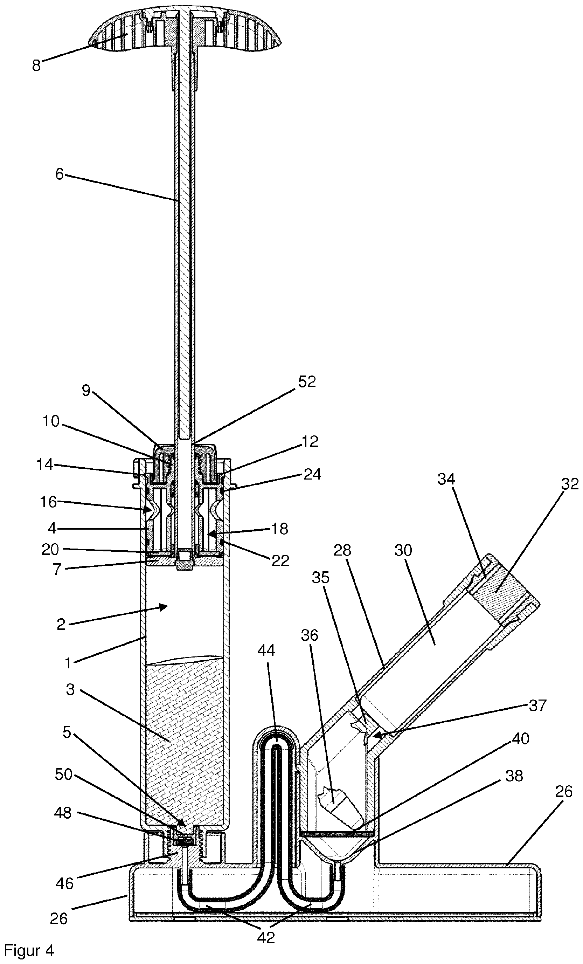

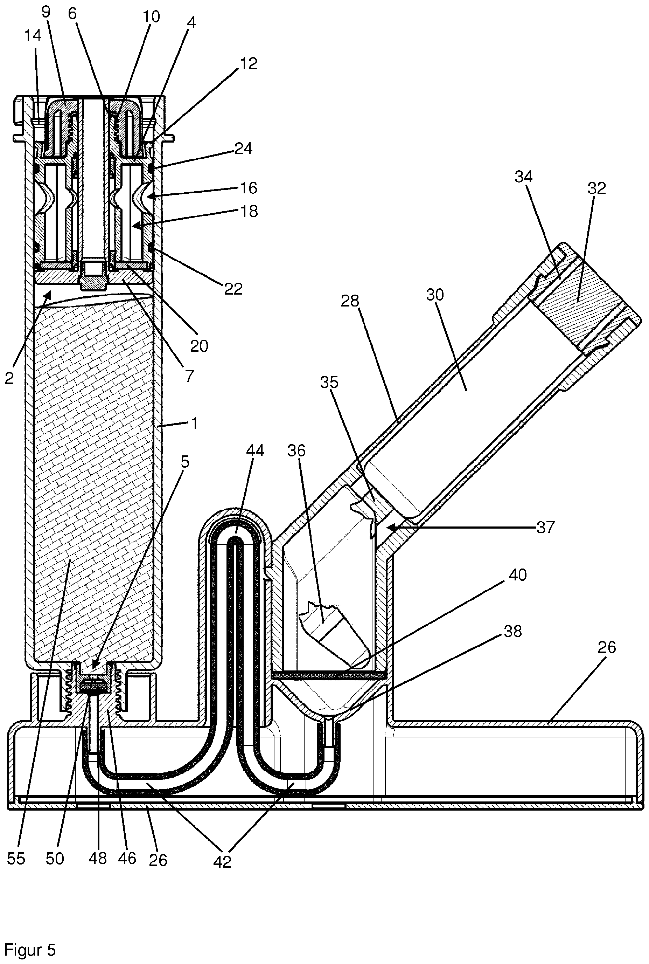

Der Behälter 28 ist mit einem Deckel 32 verschlossen, der als Stopfen in den Behälter 28 eingesteckt ist. In dem Deckel 32 sind mehrere Durchführungen 34 vorgesehen, durch die Luft nach außen abgegeben werden kann oder durch die Luft in den Behälter 28 nachströmen kann. Die Glasampulle 30 wird durch den Deckel 32 und durch eine Halterung 35 gehalten, die die Glasampulle 30 an einem Hals im Bereich eines Kopfs 36 der Glasampulle 30 hält. Durch ein Knicken des Behälters 28 kann der Kopf 36 abgebrochen werden und die Monomerflüssigkeit fließt aus der Glasampulle 30 nach unten aus. In der Halterung sind Durchführungen 37 für einen Luftaustausch vorgesehen. Wenn der Kopf 36 von der Glasampulle 30 abgebrochen wird (wie in den Figuren 4 und 5 dargestellt), fließt die Monomerflüssigkeit aus der Glasampulle 30 zu einem Trichter 38 aus, während der Kopf 36 und eventuell entstehende Splitter von einem Sieb 40 oder auch einem Filter zurückgehalten werden. Der Abstand zwischen der Halterung 35 und dem Sieb 40 ist so groß gewählt und der Behälter 28 und das Sieb 40 sind derart breit, dass der abgebrochene Kopf 36 so fallen kann, dass er sich drehen kann und seitlich zum Liegen kommt, damit möglichst keine oder nur wenig Monomerflüssigkeit zurückhalten kann. Anstelle des elastischen Behälters 28 und der Fixierung 35 des Kopfs 36 der Glasampulle, kann auch ohne weiteres ein anderer Öffnungsmechanismus verwendet werden, mit dem die Glasampulle 30 im Inneren der Vorrichtung geöffnet werden kann.

Wenn der Kopf 36 abgebrochen wurde, fließt die Monomerflüssigkeit also durch das Sieb 40 und wird von dem Trichter 38 in eine Verbindungsleitung 42 geleitet. Die Verbindungsleitung 42 bildet eine Schlaufe 44 als Siphon, die so hoch ist, dass die Monomerflüssigkeit nicht von alleine über die Schlaufe 44 hinaus in Richtung der Kartusche 1 beziehungsweise des Mischraums 2 fließen kann, wenn die Monomerflüssigkeit vollständig aus der Glasampulle 30 ausgelaufen ist. Im Bereich der Schlaufe 44 weist das Gehäuse, das durch den Standfuß 26 gebildet wird, ein Sichtfenster auf, durch den der Anwender beobachten kann, ob Monomerflüssigkeit über die Schlaufe 44 zum Mischraum 2 geleitet wird.

Die Verbindungsleitung 42 mündet in einem Stutzen 46 mit einem Außengewinde über einen pulverundurchlässigen und für die Monomerflüssigkeit durchlässigen Filter 48 und eine Düse 50 in den Mischraum 2. Der Filter 48 verhindert, dass das Knochenzementpulver 3 aus dem Mischraum 2 in die Verbindungleitung 42 gelangt und dort mit der Monomerflüssigkeit reagiert, dort aushärtet und die Verbindungsleitung 42 ungewollt verstopft. Mit der Düse 50, die wie eine Düse gemäß der US 8 662 736 B2 ausgeführt ist, wird erreicht, dass die Monomerflüssigkeit auch tiefer ins Innere des Knochenzementpulvers 3 gelangt und nicht im Bereich des Austragsöffnung 5 verklebt und ein weiteres Einleiten der Monomerflüssigkeit erschwert oder verhindert.

Der Mischstab 6 weist eine Sollbruchstelle 52 auf, die unmittelbar oberhalb der Schraubkappe 9 liegt, wenn die Schraubkappe zur Befestigung des Mischstabs 6 an dem Austragskolben 4 auf den Austragskolben 4 aufgeschraubt ist und wenn der Mischstab 6 derart weit aus dem Mischraum 2 herausgezogen ist, dass die Mischeinrichtung 7 an der Unterseite des Austragskolbens 4 anliegt, so wie dies in den Figuren 3 und 4 dargestellt ist.

Im Folgenden wird anhand der Figuren 1 bis 5 ein beispielhaftes erfindungsgemäßes Verfahren vorgestellt. Zunächst steht der Austragskolben 4, wie in den Figuren 1 und 2 gezeigt in einer Sterilisationsstellung. Die Vorrichtung wird in eine Sterilisationskammer (nicht gezeigt) gestellt und Luft wird aus der Sterilisationskammer evakuiert. Dabei tritt auch die Luft aus dem Mischraum 2 durch die Porenscheibe 20 und die Durchführung 18 des Austragskolbens 4 aus. Ebenso wird die Luft durch die Durchführungen 34 und 37 aus dem Behälter 28 und der Verbindungsleitung 42, 44 evakuiert. Anschließend wird durch die gleichen Öffnungen 16, 18, 34, 37 Ethylenoxid eingespeist und damit die Vorrichtung von Innen und außen sterilisiert. Die Vorrichtung wird anschließend aus der Sterilisationskammer entnommen.

Anschließend wird der Mischstab 6 so weit nach oben gezogen, dass die Mischeinrichtung 7 an der Unterseite des Austragskolbens 4 anliegt und mit der Schraubkappe 9 wird der Mischstab 6 mit dem Austragskolben 4 fixiert. Der Austragskolben 4 wird mit dem Mischstab 6 in die Kartusche 1 eingeschoben. Dabei verschließt sich der Mischraum 2, wenn die obere Dichtung 24 an den Innenwänden der Kartusche 1 anliegt. Der Austragskolben 4 wird bis auf das Knochenzementpulver 3 herabgedrückt. Dabei strömt die Luft beziehungsweise das Gas aus dem Mischraum 2 durch die Verbindungsleitung 42 und die Durchführungen 37, 34 aus der Vorrichtung. Dieser Zustand ist in Figur 3 gezeigt.

Durch biegen des Behälters 28 wird der Kopf 36 der Glasampulle 30 abgebrochen und die Monomerflüssigkeit fließt, wie oben beschrieben, aus der Glasampulle 30 aus, ohne über die Schlaufe 44 überzutreten. Anschließend wird der Austragskolben 4 mit dem Griff 8 und dem Mischstab 6 nach oben (weg von der Austragsöffnung 5) gezogen. Dabei entsteht im Mischraum 2 ein Unterdruck, mit dem die Monomerflüssigkeit durch die Verbindungsleitung 42, den Filter 48, die Düse 50 und die Austragsöffnung 5 in den Mischraum 2 hineingezogen, wo sie sich mit dem Knochenzementpulver 3 vermischt. Der Austragskolben 4 wird so weit nach oben gezogen, bis die Rastelemente 12 in die Gegenrastung 14 greifen und dadurch die weitere Bewegung stoppen und den Austragskolben 4 in Position halten. Diese Position ist in Figur 4 gezeigt.

Um eine bessere Durchmischung des Knochenzementpulvers 3 mit der Monomerflüssigkeit zu erreichen, wird die Schraubkappe 9 und damit das Feststellelement 9, 10 gelöst und die Mischeinrichtung 7 manuell im Mischraum 2 durch Eindrücken und Herausziehen des Mischstabs 6 in dem Mischraum 2 bewegt. Gegebenenfalls kann die Mischeinrichtung 7 auch im Mischraum 2 gedreht werden. Dabei bildet sich im Mischraum 2 die gewünschte Knochenzementpaste 55. Der Mischstab 6 wird wieder bis zum Anschlag aus dem Mischraum 2 herausgezogen, das heißt, bis die Mischeinrichtung 7 an der Unterseite des Austragskolbens 4 anliegt, und der Mischstab 6 wird wieder mit dem Befestigungselement 9, 10 an dem Austragskolben 4 fixiert. Anschließend wird der Mischstab 6 an der Sollbruchstelle 52 abgebrochen. Nun kann der Austragskolben 4 durch lösen der Rastelemente 12 wieder bewegt werden. Diese Anordnung ist in Figur 5 dargestellt.

Anschließend kann die Kartusche 1 vom Standfuß 26 abgeschraubt werden und in eine Auspressvorrichtung (nicht gezeigt) eingesetzt werden. In das Innengewinde der Kartusche 1 kann ein Austragsrohr (nicht gezeigt) eingeschraubt werden, in dem ein statischer Mischer enthalten sein kann. Mit der Auspressvorrichtung kann dann der Austragskolben 4 in Richtung der Austragsöffnung 5 vorgetrieben werden und der Knochenzement 55 aus der Austragsöffnung 5 beziehungsweise dem Austragsrohr heraus appliziert werden.

Die Knochenzementpaste 55 bildet sich auch bereits kurz nach Beginn des Einsaugens im Bereich der Düse 50, an der die Monomerflüssigkeit in den Mischraum 2 einmündet. Wenn nach einer ausreichenden Zeit die Knochenzementpaste 55 eine hinreichend zähe Konsistenz aufweist, wird die Düse 50 durch die Knochenzementpaste 55 verklebt. Dadurch wird verhindert, dass die Knochenzementpaste 55 durch die Düse 50 hindurch und in die Düse 50 hinein in Richtung der Verbindungsleitung 42 gelangt. Dadurch kann ein weiterer Monomerflüssigkeitstransfer sichergestellt werden.

Die größeren Bauteile der Vorrichtungen der beiden Ausführungsbeispiele können im Wesentlichen durch Spritzgießen aus Kunststoff gefertigt werden.

### Bezugszeichenliste

- 1: Kartusche
- 2: Mischraum
- 3: Knochenzementpulver
- 4: Austragskolben
- 5: Austragsöffnung
- 6: Mischstab
- 7: Mischflügel / Mischeinrichtung
- 8: Griff
- 9: Schraubkappe / Feststellelement
- 10: Außengewinde
- 11: Innengewinde
- 12: Rastelement
- 13: Bedienelement
- 14: Gegenrastung
- 16: Seitliche Öffnung
- 18: Durchführung
- 20: Porenscheibe
- 22: Untere Dichtung
- 24: Obere Dichtung
- 26: Fußteil
- 28: Behälter
- 30: Glasampulle / Monomerflüssigkeitsbehälter
- 32: Deckel
- 34: Durchführung
- 35: Halterung
- 36: Kopf der Ampulle
- 37: Durchführung
- 38: Trichter
- 40: Sieb
- 42: Verbindungsleitung
- 44: Schlaufe / Siphon
- 46: Stutzen mit Außengewinde
- 48: Flüssigkeitsdurchlässiger, pulverundurchlässiger Filter
- 50: Düse
- 52: Sollbruchstelle
- 55: Knochenzementpaste

## Patentansprüche

1. Vorrichtung zum Mischen eines Knochenzements (55), die Vorrichtung aufweisend
zumindest eine Kartusche (1), wobei die Kartusche (1) in ihrem Inneren einen zylindrischen Mischraum (2) aufweist,
einen Austragskolben (4), der im zylindrischen Mischraum (2) in Längsrichtung beweglich ist und der gegen die Wände des Mischraums (2) abgedichtet ist, eine Mischeinrichtung (7) zum Durchmischen des Inhalts des Mischraums (2), die im Inneren des Mischraums (2) angeordnet ist, wobei die Mischeinrichtung (7) über einen Mischstab (6) von außen bedienbar ist und wobei der Mischstab (6) durch eine Durchführung in dem Austragskolben (4) drehbar und in Längsrichtung verschiebbar durch den Austragskolben (4) geführt ist, wobei die Kartusche (1) eine dem Austragskolben (4) gegenüberliegende Austragsöffnung (5) aufweist, wobei im Mischraum (2) zwischen dem Austragskolben (4) und der Austragsöffnung (5) eine erste Komponente (3) des Knochenzements (55) enthalten ist, wobei eine Monomerflüssigkeit als zweite Komponente des Knochenzements (55) durch die Austragsöffnung (5) in den Mischraum (2) einleitbar ist, wobei
die Vorrichtung ein Feststellelement (9, 10) aufweist, mit dem der Mischstab (6) mit dem Austragskolben (4) derart fixierbar oder fixiert ist, dass der Austragskolben (4) mittels des Mischstabs (6) in dem Mischraum (2) in Längsrichtung bewegbar ist, **dadurch gekennzeichnet, dass** der Austragskolben (4) eine gasundurchlässige Oberseite aufweist, die von der Austragsöffnung (5) abgewandt ist, und eine gasdurchlässige und für Pulver-Partikel undurchlässige Unterseite aufweist, die der Austragsöffnung (5) zugewandt ist, wobei die gasdurchlässige Unterseite über zumindest eine Durchführung (18) im Austragskolben (4) mit zumindest einer seitlichen Öffnung (16) in der Seitenfläche des Austragskolbens (4) verbunden ist, so dass der Mischraum (2) gasdurchlässig mit der Umgebung der Kartusche (1) verbunden ist, wenn die zumindest eine Öffnung (16) in der Seitenfläche des Austragskolbens (4) nicht durch die Innenwand des Mischraums (2) der Kartusche (1) verdeckt ist.

2. Vorrichtung nach Anspruch 1, **dadurch gekennzeichnet, dass**
der Mischstab (6) mit dem Feststellelement (9, 10) lösbar mit dem Austragskolben (4) fixierbar oder fixiert ist.

3. Vorrichtung nach einem der vorangehenden Ansprüche, **dadurch gekennzeichnet, dass**
zumindest ein Rastelement (12) an dem Austragskolben (4) vorgesehen ist, das derart in wenigstens eine Gegenrastung (14) an der Kartusche (1) greift oder dazu geeignet ist derart in wenigstens eine Gegenrastung (14) zu greifen, dass der Austragskolben (4) nicht mehr über die zumindest eine Rastposition hinaus in Richtung von der Austragsöffnung (5) weg bewegt werden kann, wobei vorzugsweise das zumindest eine Rastelement (12) an einer seitlichen Mantelfläche des Austragskolbens (4) angeordnet ist und die wenigstens eine Gegenrastung (14) an der Innenwand des Mischraums (2) angeordnet ist.

4. Vorrichtung nach Anspruch 3, **dadurch gekennzeichnet, dass**
wenigstens eines der zumindest einen Rastelemente (12) über zumindest ein Bedienelement (13) von außen bedienbar ist, wobei vorzugsweise das Bedienelement (13) mit dem wenigstens einen der zumindest einen Rastelemente (12) verbunden ist und über den oberen Rand der Kartusche (1) hinausragt, wobei der obere Rand der Kartusche (1) der Austragsöffnung (5) gegenüber liegt, wobei vorzugsweise das Bedienelement (13) einteilig mit dem wenigstens einen der zumindest einen Rastelemente (12) verbunden ist.

5. Vorrichtung nach einem der vorangehenden Ansprüche, **dadurch gekennzeichnet, dass**
der Austragskolben (4) zwischen der gasundurchlässigen Oberseite des Austragskolbens (4) und der zumindest einen seitlichen Öffnung (16) eine obere umlaufende Dichtung (24) sowie zwischen der gasdurchlässigen Unterseite und der zumindest einen seitlichen Öffnung (16) eine untere umlaufende Dichtung (22) aufweist, die den Austragskolben (4) gegen die Innenwand des Mischraums (2) abdichten oder die dazu geeignet sind, den Austragskolben (4) gegen die Innenwand des Mischraums (2) abzudichten, wenn der Austragskolben (4) ausreichend tief in Richtung der Austragsöffnung (5) in die Kartusche (1) eingeschoben ist.

6. Vorrichtung nach einem der Ansprüche 3 bis 5, **dadurch gekennzeichnet, dass** zumindest zwei Rastpositionen für den Austragskolben (4) vorgesehen sind, wobei die zumindest eine seitliche Öffnung (16) in einer ersten Rastposition offen liegt und in einer zweiten Rastposition die zumindest eine seitliche Öffnung (16) verdeckt ist.

7. Vorrichtung nach Anspruch 6, **dadurch gekennzeichnet, dass** in der ersten Rastposition der Austragskolben (4) nur durch die untere umlaufende Dichtung (22) gegen den Mischraum (2) abgedichtet ist und in der zweiten Rastposition der Austragskolben (4) durch die untere und obere Dichtung (24) gegen den Mischraum (2) abgedichtet ist.

8. Vorrichtung nach einem der vorangehenden Ansprüche, **dadurch gekennzeichnet, dass**
das Feststellelement (9, 10) eine Schraubkappe (9) ist, die den Mischstab (6) umschießt und die derart auf oder in ein Gegengewinde (10) an dem Austragskolben (4) schraubbar ist, dass sich die Schraubkappe (9) oder die Durchführung durch den Austragskolben (4) derart an den Mischstab (6) presst, dass der Mischstab (6) gegen den Austragskolben (4) fixiert ist, oder
das Feststellelement (9, 10) wenigstens ein Zapfen ist, der durch zumindest eine Durchführung im Mischstab (6) zu stecken ist oder der in zumindest eine Vertiefung im Mischstab (6) zu stecken ist, so dass der Mischstab (6) nicht mehr in den Mischraum (2) einzuführen ist, wobei vorzugsweise die zumindest eine Durchführung oder die zumindest eine Vertiefung derart positioniert ist, dass die Mischeinrichtung (7) auf der der Austragsöffnung (5) zugewandten Unterseite des Austragskolbens (4) anliegt, wenn der Mischstab (6) mit dem wenigstens einen Zapfen gegen den Austragskolben (4) fixiert ist.

9. Vorrichtung nach einem der vorangehenden Ansprüche, **dadurch gekennzeichnet, dass**
das freie Volumen im Mischraum (2), das nicht von der ersten Komponente (3) und dem Austragskolben (4) eingenommen wird, wenn der Austragskolben (4) maximal weit in Richtung von der Austragsöffnung (5) weg in der Kartusche (1) angeordnet ist, mindestens so groß ist wie das Volumen der einzuleitenden Monomerflüssigkeit und aller zwischen der Monomerflüssigkeit und dem Mischraum (2) vorgesehenen Verbindungsleitungen (42).

10. Vorrichtung zum Lagern der Ausgangskomponenten eines Knochenzements (55) und zum Mischen des Knochenzements (55) nach einem der vorangehenden Ansprüche, insbesondere Full-Prepacked-Zementiervorrichtung, **dadurch gekennzeichnet, dass**
eine Verbindungsleitung (42) vorgesehen ist, die mit der Austragsöffnung (5) verbunden ist und die den Mischraum (2) flüssigkeitsdurchlässig mit einem Behälter (28) verbindet, wobei in dem Behälter (28) ein Monomer-Behälter (30) mit einer Monomerflüssigkeit darin einsetzbar ist oder enthalten ist, wobei der Monomer-Behälter (30) in der Vorrichtung zu öffnen ist und die Monomerflüssigkeit durch die Verbindungsleitung (42) in den Mischraum (2) einzusaugen ist, indem durch eine Bewegung des Austragskolbens (4) von der Austragsöffnung (5) weg im Mischraum (2) ein Unterdruck erzeugt wird.

11. Vorrichtung nach Anspruch 10, **dadurch gekennzeichnet, dass**
in dem Behälter (28) zumindest eine Durchführung (34) vorgesehen ist, wobei die zumindest eine Durchführung (34) den Behälter (28) mit der Umgebung gasdurchlässig verbindet.

12. Vorrichtung nach Anspruch 10 oder 11, **dadurch gekennzeichnet, dass**
die Vorrichtung eine Öffnungseinrichtung aufweist, mit der der Monomer-Behälter (30) in dem Behälter (28) zu öffnen ist, vorzugsweise ohne die Vorrichtung dabei zu öffnen oder ohne den Behälter (28) dabei zu öffnen.

13. Verfahren zum Herstellen eines Knochenzements (55) aus einer ersten Komponente (3) und einer Monomer-Flüssigkeit als zweiter Komponente, insbesondere mit einer Vorrichtung nach einem der Ansprüche 1 bis 12, **dadurch gekennzeichnet, dass**
ein Mischstab (6) mit Hilfe eines Feststellelements (9, 10) mit einem Austragskolben (4) fixiert wird oder fixiert ist,
der Austragskolben (4) von einer Austragsöffnung (5) weg bewegt wird und sich dadurch ein Unterdruck in einem Mischraum (2) bildet, wobei vorzugsweise der Austragskolben (4) manuell mit Hilfe des Mischstabs (6) bewegt wird,
mit dem Unterdruck eine Monomerflüssigkeit in den Mischraum (2) gesaugt wird,
der Mischstab (6) von dem Austragskolben (4) gelöst wird und die erste Komponente (3) des Knochenzements (55) und die Monomerflüssigkeit im Mischraum (2) mit der Mischeinrichtung (7) durch manuelles Bewegen eines Mischstabs (6) zu einer Knochenzementpaste (55) gemischt wird, wobei
der Mischraum (2) zunächst über den Austragskolben (4) gasdurchlässig mit der Umgebung einer Kartusche (1) verbunden ist, wobei die Vorrichtung einschließlich des Mischraums (2) durch Evakuieren und Einleiten eines sterilisierenden Gases, insbesondere von Ethylenoxid, sterilisiert wird, anschließend der Austragskolben (4) in Richtung der Austragsöffnung (5) bewegt wird, so dass die gasdurchlässigen Öffnungen (16) im Austragskolben (4) durch die Innenwand des Mischraums (2) verdeckt werden, und anschließend der Austragskolben (4) derart tief in den Mischraum (2) eingeschoben wird, dass Gas aus dem Mischraum (2) durch die Austragsöffnung (5) herausgedrückt wird, bevor der Austragskolben (4) von der Austragsöffnung (5) weg bewegt wird.

14. Verfahren nach Anspruch 13, **dadurch gekennzeichnet, dass** anschließend der Monomerbehälter (30) in dem Behälter (28) der Vorrichtung geöffnet
wird, bevor der Austragskolben (4) von der Austragsöffnung (5) weg bewegt
wird.

15. Verfahren nach Anspruch 13 oder 14, **dadurch gekennzeichnet, dass**
der Austragskolben (4) zumindest so weit in Richtung der Austragsöffnung (5) in den Mischraum (2) eingeschoben wird, dass der dabei überfahrene Hubraum mindestens so groß ist, wie das Volumen der einzuleitenden Monomerflüssigkeit und aller Verbindungsleitungen (42), durch die die Monomerflüssigkeit in den Mischraum (2) geleitet wird.

16. Verfahren nach einem der Ansprüche 13 bis 15, **dadurch gekennzeichnet, dass** bei einem Einschieben des Austragskolbens (4) in den Mischraum (2) Gas aus dem Mischraum (2) durch die Austragsöffnung (5) und durch zumindest eine Durchführung (34) in einem Behälter (28) für die Monomerflüssigkeit aus der Vorrichtung herausgedrückt wird und/oder wenn die Monomerflüssigkeit in den Mischraum (2) gesaugt wird, Luft durch zumindest eine Durchführung (34) in einem Behälter (28) für die Monomerflüssigkeit in den Behälter (28) nachströmt.

## Claims

1. A device for mixing a bone cement (55), the device comprising
at least one cartridge (1), wherein the cartridge (1) comprises in its interior a cylindrical mixing chamber (2),
a discharge piston (4) which can be moved in the cylindrical mixing chamber (2) in the longitudinal direction and which is sealed against the walls of the mixing chamber (2),
a mixing device (7) for mixing the content of the mixing chamber (2), which is arranged in the interior of the mixing chamber (2), wherein the mixing device (7) can be operated externally via a mixing rod (6) and wherein the mixing rod (6) is guided through a passage in the discharge piston (4) in such a manner that it can be rotated and displaced in the longitudinal direction by the discharge piston (4), wherein
the cartridge (1) comprises a discharge opening (5) opposite the discharge piston (4), wherein in the mixing chamber (2) a first component (3) of the bone cement (55) is contained between the discharge piston (4) and the discharge opening (5), wherein a monomer fluid as a second component of the bone cement (55) is introducable through the discharge opening (5) into the mixing chamber (2), wherein
the device comprises a locking element (9, 10) with which the mixing rod (6) is affixable or is affixed to the discharge piston (4) in such a manner that the discharge piston (4) can be moved by means of the mixing rod (6) in the mixing chamber (2) in the longitudinal direction, **characterized in that**
the discharge piston (4) comprises a gas impermeable upper side which faces away from the discharge opening (5) and a gas permeable underside, which is impermeable for powder particles, which faces towards the discharge opening (5), wherein the gas permeable underside is connected via at least one passage (18) in the discharge piston (4) with at least one side opening (16) in the side surface of the discharge piston (4), so that the mixing chamber (2) is gas-permeably connected with the environment of the cartridge (1) when the at least one opening (16) in the side surface of the discharge piston (4) is not covered by the inner wall of the mixing chamber (2) of the cartridge (1).

2. The device according to claim 1, **characterized in that**
the mixing rod (6) with the locking element (9, 10) is detachably affixable or is detachably affixed to the discharge piston (4).

3. The device according to any one of the preceding claims, **characterized in that** at least one latching element (12) is provided on the discharge piston (4), which grips into at least one counter-latching means (14) on the cartridge (1) in such a manner, or is suitable for gripping into at least one counter-latching means (14) in such a manner, that the discharge piston (4) can no longer be moved in the direction away from the discharge opening (5) beyond the at least one latching position, wherein preferably the at least one latching element (12) is arranged on a side sheath surface of the discharge piston (4) and the at least one counter-latching means (14) is arranged on the inner wall of the mixing chamber (2).

4. The device according to claim 3, **characterized in that**
at least one of the at least one latching elements (12) can be operated externally via at least one operating element (13), wherein preferably, the operating element (13) is connected to the at least one of the at least one latching elements (12) and protrudes over the upper edge of the cartridge (1), wherein the upper edge of the cartridge (1) lies opposite the discharge opening (5), wherein preferably, the operating element (13) is connected as a single piece with the at least one of the at least one latching elements (12).

5. The device according to any one of the preceding claims, **characterized in that** the discharge piston (4) comprises an upper circumferential seal (24) between the gas impermeable upper side of the discharge piston (4) and the at least one side opening (16) and a lower circumferential seal (22) between the gas permeable underside and the at least one side opening (16), which seal the discharge piston (4) against the inner wall of the mixing chamber (2), or are suitable for sealing the discharge piston (4) against the inner wall of the mixing chamber (2), when the discharge piston (4) has been inserted sufficiently deeply into the cartridge (1) in the direction of the discharge opening (5).

6. The device according to any one of claims 3 to 5, **characterized in that**
at least two latching positions are provided for the discharge piston (4), wherein the at least one side opening (16) lies exposed in a first latching position and in a second latching position, the at least one side opening (16) is covered.

7. The device according to claim 6, **characterized in that**
in the first latching position the discharge piston (4) is only sealed against the mixing chamber (2) by the lower circumferential seal (22), and in the second latching position, the discharge piston (4) is sealed against the mixing chamber (2) by the upper and lower seal (24).

8. The device according to any one of the preceding claims, **characterized in that** the affixing element (9, 10) is a screw cap (9) which encloses the mixing rod (6) and which can be screwed onto or into a counter-thread (10) on the discharge piston (4) in such a manner that the screw cap (9) or the passage through the discharge piston (4) presses onto the mixing rod (6) in such a manner that the mixing rod (6) is affixed against the discharge piston (4), or
the affixing element (9, 10) is at least one pin which is to be inserted through at least one passage in the mixing rod (6), or which is to be inserted into at least one recess in the mixing rod (6), so that the mixing rod (6) can no longer be inserted into the mixing chamber (2), wherein preferably, the at least one passage or the at least one recess is positioned in such a manner that the mixing device (7) lies on the underside of the discharge piston (4) facing towards the discharge opening (5) when the mixing rod (6) is affixed against the discharge piston (4) with the at least one pin.

9. The device according to any one of the preceding claims, **characterized in that** the free volume in the mixing chamber (2) which is not taken up by the first component (3) and the discharge piston (4) when the discharge piston (4) is arranged in the cartridge (1) at a maximum distance in the direction away from the discharge opening (5) is at least as large as the volume of the monomer fluid to be introduced and all connection lines (42) provided between the monomer fluid and the mixing chamber (2).

10. A device for storing the initial components of a bone cement (55) and for mixing the bone cement (55) according to any one of the preceding claims, in particular a full-prepacked cementing device, **characterized in that**
a connection line (42) is provided which is connected to the discharge opening (5) and which connects the mixing chamber (2) in a fluid permeable manner with a vessel (28), wherein in the vessel (28), a monomer vessel (30) with a monomer fluid contained therein can be inserted or is contained, wherein the monomer vessel (30) should be opened in the device and the monomer fluid should be suctioned into the mixing chamber (2) through the connection line (42) by an underpressure which is generated in the mixing chamber (2) by a movement of the discharge piston (4) away from the discharge opening (5).

11. The device according to claim 10, **characterized in that**
in the container (28), at least one passage (34) is provided, wherein the at least one passage (34) connects the vessel (28) with the environment in a gas permeable manner.

12. The device according to claim 10 or 11, **characterized in that**
the device comprises an opening device with which the monomer vessel (30) should be opened in the vessel (28), preferably without hereby opening the device or without hereby opening the vessel (28).

13. A method for producing a bone cement (55) from a first component (3) and a monomer fluid as a second component, in particular with a device according to any one of claims 1 to 12, **characterized in that**
a mixing rod (6) is affixed, or is already affixed, with the aid of a locking element (9, 10) to a discharge piston (4),
the discharge piston (4) is moved away from a discharge opening (5) and as a result, an underpressure forms in a mixing chamber (2), wherein preferably, the discharge piston (4) is moved manually with the aid of the mixing rod (6),
with the underpressure, a monomer fluid is suctioned into the mixing chamber (2),
the mixing rod (6) is detached from the discharge piston (4) and the first component (3) of the bone cement (55) and the monomer fluid are mixed in the mixing chamber (2) with a mixing device (7) through manual movement of the mixing rod (6) to form a bone cement paste (55), wherein
the mixing chamber (2) is initially connected to the environment of a cartridge (1) via the discharge piston (4), wherein the device including the mixing chamber (2) is sterilised by the evacuation and introduction of a sterilising gas, in particular ethylene oxide,
subsequently, the discharge piston (4) is moved in the direction of the discharge opening (5), so that the gas permeable openings in the discharge piston (4) are covered by the inner wall of the mixing chamber (2), and
subsequently, the discharge piston (4) is inserted sufficiently deeply into the mixing chamber (2) that gas is pressed out of the mixing chamber (2) through the discharge opening (5), before the discharge piston (4) is moved away from the discharge opening (5).

14. The method according to claim 13, **characterized in that**
the monomer vessel (30) in the vessel (28) of the device is then opened before the discharge piston (4) is moved away from the discharge opening (5).

15. The method according to claim 13 or 14, **characterized in that**
the discharge piston (4) is inserted in the direction of the discharge opening (5) into the mixing chamber (2) at least far enough that the capacity travelled over during the process is at least as large as the volume of the monomer fluid to be introduced and all connection lines (42) via which the monomer fluid is guided into the mixing chamber (2).

16. The method according to any one of claims 13 to 15, **characterized in that** when the discharge piston (4) is pushed into the mixing chamber (2), gas from the mixing chamber (2) is pressed out of the device through the discharge opening (5) and through at least one passage (34) in a vessel (28) for the monomer fluid and/or when the monomer fluid is suctioned into the mixing chamber (2), air then flows through at least one passage (34) in a vessel (28) for the monomer fluid into the vessel (28).

## Revendications

1. Dispositif de mélange d'un ciment osseux (55), le dispositif présentant
au moins une cartouche (1), où la cartouche (1) présente une chambre de mélange (2) cylindrique dans sa partie intérieure,
un piston d'évacuation (4) qui est mobile dans la direction longitudinale dans la chambre de mélange (2) cylindrique et qui est rendu étanche au niveau des parois de la chambre de mélange (2),
un dispositif de mélange (7) permettant de mélanger le contenu de la chambre de mélange (2) qui est disposé à l'intérieur de la chambre de mélange (2), où le dispositif de mélange (7) peut être actionné de l'extérieur par le biais d'une tige de mélange (6) et où la tige de mélange (6) peut être mise en rotation à travers une conduite de passage dans le piston d'évacuation (4) et est menée en pouvant être déplacée dans la direction longitudinale à travers le piston d'évacuation (4), où la cartouche (1) présente un orifice de sortie (5) se situant en face du piston d'évacuation (4), où un premier composant (3) du ciment osseux (55) est contenu dans la chambre de mélange (2) entre le piston d'évacuation (4) et l'orifice de sortie (5), où un liquide de monomère servant de second composant du ciment osseux (55) peut être introduit dans la chambre de mélange (2) par l'orifice de sortie (5), où
le dispositif présente un élément de réglage (9, 10) avec lequel la tige de mélange (6) peut être fixée ou est fixée avec le piston d'évacuation (4) de sorte que le piston d'évacuation (4) peut être déplacé dans la direction longitudinale dans la chambre de mélange (2) au moyen de la tige de mélange (6), **caractérisé en ce que**
le piston d'évacuation (4) présente un côté supérieur imperméable aux gaz qui est opposé à l'orifice de sortie (5) et présente un côté inférieur perméable aux gaz et imperméable pour des particules de poudre, qui est orienté vers l'orifice de sortie (5), où le côté inférieur perméable aux gaz est relié avec au moins un orifice latéral (16) dans la surface latérale du piston d'évacuation (4) par le biais d'au moins une conduite de passage (18) dans le piston d'évacuation (4), de sorte que la chambre de mélange (2) est reliée avec l'environnement de la cartouche (2) en étant perméable aux gaz lorsque l'au moins un orifice (16) dans la surface latérale du piston d'évacuation (4) n'est pas recouvert par la paroi intérieure de la chambre de mélange (2) de la cartouche (1).

2. Dispositif selon la revendication 1, **caractérisé en ce que**
la tige de mélange (6) peut être fixée ou est fixée avec l'élément de réglage (9, 10) de manière amovible avec le piston d'évacuation (4).

3. Dispositif selon l'une des revendications précédentes, **caractérisé en ce**
**qu'**au moins un élément de blocage (12) est prévu sur le piston d'évacuation (4) qui se met en prise dans un élément de blocage complémentaire (14) sur la cartouche (1) ou qui est approprié pour se mettre en prise dans au moins un tel élément de blocage complémentaire (14) que le piston d'évacuation (4) ne peut plus être déplacé en direction de l'orifice de sortie (5) au-delà de l'au moins une position de blocage, où, de préférence, l'au moins un élément de blocage (12) est disposé dans une surface d'enveloppe latérale du piston d'évacuation (4) et l'au moins un blocage complémentaire (14) est disposé dans la paroi intérieure de la chambre de mélange (2).

4. Dispositif selon la revendication 3, **caractérisé en ce**
**qu'**au moins l'un de l'au moins un élément de blocage (12) peut être actionné de l'extérieur par le biais d'au moins un élément d'actionnement (13), où de préférence, l'élément d'actionnement (13) est relié avec l'au moins un de l'au moins un élément de blocage (12) et dépasse par-dessus le bord supérieur de la cartouche (1), où le bord supérieur de la cartouche (1) se situe en face de l'orifice de sortie (5), où, de préférence, l'élément d'actionnement (13) est relié d'un seul tenant avec l'au moins un de l'au moins un élément de blocage (12).

5. Dispositif selon l'une des revendications précédentes, **caractérisé en ce que**
le piston d'évacuation (4) présente un joint circonférentiel supérieur (24) entre le côté supérieur du piston d'évacuation (4) imperméable aux gaz et l'au moins un orifice latéral (16), ainsi qu'un joint circonférentiel inférieur (22) entre le côté inférieur perméable aux gaz et l'au moins un orifice latéral (16), qui rendent la paroi intérieure de la chambre de mélange (2) étanche vis-à-vis du piston d'évacuation (4) ou qui sont appropriés pour rendre le piston d'évacuation (4) étanche vis-à-vis de la paroi intérieure de la chambre de mélange (2) lorsque le piston d'évacuation (4) est inséré suffisamment loin dans la cartouche (1) en direction de l'orifice de sortie (5).

6. Dispositif selon l'une des revendications 3 à 5, **caractérisé en ce**
**qu'**au moins deux positions de blocage sont prévues pour le piston d'évacuation (4), où l'au moins un orifice latéral (16) est ouvert dans une première position de blocage et l'au moins un orifice latéral (16) est recouvert dans une deuxième position de blocage.

7. Dispositif selon la revendication 6, **caractérisé en ce que**
dans la première position de blocage, le piston d'évacuation (4) n'est rendu étanche vis-à-vis de la chambre de mélange (2) que par le joint circonférentiel inférieur (22) et le piston d'évacuation (4) est rendu étanche vis-à-vis de la chambre de mélange (2) par le joint inférieur et le joint supérieur (24) dans la deuxième position de blocage.

8. Dispositif selon l'une des revendications précédentes, **caractérisé en ce que** l'élément de réglage (9, 10) est un capuchon fileté (9) qui entoure la tige de mélange (6) et qui peut être vissé sur ou dans un filetage complémentaire (10) sur le piston d'évacuation (4) de sorte que le capuchon fileté (9) ou la conduite de passage presse sur la tige de mélange (6) à travers le piston d'évacuation (4) de telle manière que la tige de mélange (6) est fixée contre le piston d'évacuation (4), ou
l'élément de réglage (9, 10) est au moins un bouchon qui est à insérer par l'au moins une conduite de passage dans la tige de mélange (6) ou qui est à insérer dans au moins une cavité dans la tige de mélange (6), de sorte que la tige de mélange (6) n'est plus à insérer dans la chambre de mélange (2), où de préférence l'au moins une conduite de passage ou l'au moins une cavité est positionné(e) de sorte que le dispositif de mélange (7) se plaque sur le côté inférieur du piston d'évacuation (4) orienté vers l'orifice de sortie (5) lorsque la tige de mélange (6) est fixée avec l'au moins un bouchon contre le piston d'évacuation (4).

9. Dispositif selon l'une des revendications précédentes, **caractérisé en ce que**
le volume libre dans la chambre de mélange (2) qui n'est pas occupé par le premier composant (3) et le piston d'évacuation (4) lorsque le piston d'évacuation (4) est disposé dans la cartouche (1) au maximum éloigné en direction de l'orifice d'évacuation (5), est au moins aussi important que le volume du liquide de monomère à introduire et de toutes les conduites de liaison (42) prévues entre le liquide de monomère et la chambre de mélange (2).

10. Dispositif de stockage des composants de départ d'un ciment osseux (55) et de mélange du ciment osseux (55) selon l'une des revendications précédentes, notamment dispositif de cimentation complet pré-emballé, **caractérisé en ce**
**qu'**une conduite de liaison (42) est prévue qui est reliée avec l'orifice de sortie (5) et qui relie de manière perméable aux liquides la chambre de mélange (2) avec un récipient (28), où on peut insérer dans le récipient (28) ou est contenu un récipient de monomère (30) avec un liquide de monomère, où le récipient de monomère (30) doit être ouvert dans le dispositif et le liquide de monomère est à aspirer dans la chambre de mélange (2) par la conduite de liaison (42) par le fait qu'un pression négative est générée par un déplacement du piston d'évacuation (4) dans la chambre de mélange (2) s'éloignant de l'orifice de sortie (5).

11. Dispositif selon la revendication 10, **caractérisé en ce**
**qu'**au moins une conduite de passage (34) est prévue dans le récipient (28), où l'au moins une conduite de passage (34) relie le récipient (28) avec l'environnement de manière perméable aux gaz.

12. Dispositif selon la revendication 10 ou la revendication 11, **caractérisé en ce que**
le dispositif présente un dispositif d'ouverture avec lequel on doit ouvrir le récipient de monomère (30) dans le récipient (28), de préférence sans ouvrir le dispositif ce faisant ou sans ouvrir le récipient (28) ce faisant.

13. Procédé de fabrication d'un ciment osseux (55) à partir d'un premier composant (3) et d'un liquide de monomère servant de second composant, en particulier, avec un dispositif selon l'une des revendications 1 à 12, **caractérisé en ce**
**qu'**une tige de mélange (6) se trouve fixée ou est fixée à l'aide d'un élément de réglage (9, 10) avec un piston d'évacuation (4),
**que** le piston d'évacuation (4) est éloigné d'un orifice de sortie (5) et qu'ainsi il se forme une pression négative dans une chambre de mélange (2), où le piston d'évacuation (4) est de préférence actionné manuellement à l'aide de la tige de mélange (6),
**qu'**un liquide de monomère est aspiré dans la chambre de mélange (2) avec la pression négative,
**que** la tige de mélange (6) est détachée du piston d'évacuation (4) et que le premier composant (3) du ciment osseux (55) et le liquide de monomère sont mélangés dans la chambre de mélange (2) avec le dispositif de mélange (7) par un déplacement manuel d'une tige de mélange (6) pour donner une pâte de ciment osseux (55), où la chambre de mélange (2) est d'abord reliée avec l'environnement d'une cartouche (1) de manière perméable aux gaz par le biais du piston d'évacuation (4), où le dispositif, y compris la chambre de mélange (2), sont stérilisés par l'évacuation et l'introduction d'un gaz de stérilisation, notamment d'oxyde d'éthylène,
ensuite, le piston d'évacuation (4) est déplacé en direction de l'orifice de sortie (5) de sorte que les orifices (16) perméables aux gaz dans le piston d'évacuation (4) sont recouverts par la paroi intérieure de la chambre de mélange (2), et
ensuite le piston d'évacuation (4) est inséré dans la chambre de mélange (2) aussi loin que du gaz est pressé hors de la chambre de mélange (2) par l'orifice de sortie (5) avant que le piston d'évacuation (4) ne soit déplacé de l'orifice de sortie (5).

14. Procédé selon la revendication 13, **caractérisé en ce**
**qu'**ensuite le récipient de monomère (30) est ouvert dans le récipient (28) du dispositif avant que le piston d'évacuation (4) ne soit déplacé de l'orifice de sortie (5).

15. Procédé selon la revendication 13 ou la revendication 14, caractérisé en ce
le piston d'évacuation (4) est inséré au moins aussi loin dans la chambre de mélange (2) en direction de l'orifice de sortie (5) que l'espace ainsi parcouru est aussi important que le volume du liquide de monomère à introduire et de toutes les conduites de liaison (42) par lesquelles le liquide de monomère est introduit dans la chambre de mélange (2).

16. Procédé selon l'une des revendications 13 à 15, **caractérisé en ce que**
lors d'une insertion du piston d'évacuation (4) dans la chambre de mélange (2), du gaz est pressé hors du dispositif dans la chambre de mélange (2) par l'orifice de sortie (5) et par au moins une conduite de passage (34) dans un récipient (28) destiné au liquide de monomère et/ou lorsque le liquide de monomère est aspiré dans la chambre de mélange (2), de l'air s'écoule dans le récipient (28) par au moins une conduite de passage (34) dans un récipient (28) destiné au liquide de monomère.
